# FASCICULE DE BREVET EUROPEEN

(11) **EP 1 292 671 B1**
(45) Date de publication et mention de la délivrance du brevet: **18.11.2009**
(21) Numéro de dépôt: 01943584.1
(22) Date de dépôt: 07.06.2001
(51) Int. Cl.: C12N 5/08, C12N 5/10, A61K 48/00, A61P 9/00, A61P 19/00, A61P 21/00

(54) **PROCEDE D'OBTENTION DE POPULATIONS CELLULAIRES CARACTERISEES D'ORIGINE MUSCULAIRE ET UTILISATIONS**
VERFAHREN ZUR GEWINNUNG VON MUSKELNABSTAMMENDEN ZELLPOPULATIONEN UND DEREN VERWENDUNG
METHOD FOR OBTAINING CHARACTERISED MUSCLE-DERIVED CELL POPULATIONS AND USES

(30) Priorité: 07.06.2000 FR 0007304
(43) Date de publication de la demande: 19.03.2003
(73) Titulaire: ASSISTANCE PUBLIQUE, HOPITAUX DE PARIS, 75100 Paris (FR); INSTITUT NATIONAL DE LA SANTE ET DE LA RECHERCHE MEDICALE (INSERM), 75654 Paris Cédex 13 (FR); ASSOCIATION FRANCAISE CONTRE LES MYOPATHIES, F-75013 Paris (FR)
(72) Inventeur: VILQUIN, Jean-Thomas, F-77500 Chelles (FR); MAROLLEAU, Jean-Pierre, F-94170 le Perreux (FR); TREMBLAY, Jacques, Stoneham, Quebec G0A 4P0 (CA); ROBERT, Isabelle, F-93400 Saint-Ouen (FR); TERNAUX, Brigitte, F-78450 Villepreux (FR)
(74) Mandataire: Desaix, Anne
(86) Numéro de dépôt international: PCT/FR2001/001768
(87) Numéro de publication internationale: WO 2001/094555

(56) Documents cités:
- WO-A-99/56785
- US-A- 5 750 397
- LEQUERICA J ET AL.: "in vitro proliferation, differentiation and immuno-magnetic bead purificationof human myoblasts" ANNALS OF TRANSPLANTION, vol. 4, - 1999 pages 103-108, XP000982504 cité dans la demande
- WEBSTER C ET AL: "ISOLATION OF HUMAN MYOBLASTS WITH THE FLUORESCENCE-ACTIVATED CELL SORTER" EXPERIMENTAL CELL RESEARCH, vol. 174, no. 1, 1988, pages 252-265, XP000982441 ISSN: 0014-4827 cité dans la demande
- GUSSONI E ET AL: "DYSTROPHIN EXPRESSION IN THE MDX MOUSE RESTORED BY STEM CELL TRANSPLANTATION" NATURE,GB,MACMILLAN JOURNALS LTD. LONDON, vol. 401, 23 septembre 1999 (1999-09-23), pages 390-394, XP000867084 ISSN: 0028-0836 cité dans la demande
- JACKSON KATHYJO ANN ET AL: "Hematopoietic potential of stem cells isolated from murine skeletal muscle." PROCEEDINGS OF THE NATIONAL ACADEMY OF SCIENCES OF THE UNITED STATES, vol. 96, no. 25, 7 décembre 1999 (1999-12-07), pages 14482-14486, XP002161311 Dec. 7, 1999 ISSN: 0027-8424 cité dans la demande
- QU ZHUQING ET AL: "Development of approaches to improve cell survival in myoblast transfer therapy." JOURNAL OF CELL BIOLOGY, vol. 142, no. 5, pages 1257-1267, XP002161312 ISSN: 0021-9525 cité dans la demande
- HUARD J ET AL: "HUMAN MYOBLAST TRANSPLANTATION PRELIMINARY RESULTS OF 4 CASES" MUSCLE & NERVE, vol. 15, no. 5, 1992, pages 550-560, XP000982434 ISSN: 0148-639X cité dans la demande
- YOUNG HENRY E ET AL: "Human pluripotent and progenitor cells display cell surface cluster differentiation markers CD10, CD13, CD56, and MHC Class-I." PROCEEDINGS OF THE SOCIETY FOR EXPERIMENTAL BIOLOGY AND MEDICINE, vol. 221, no. 1, mai 1999 (1999-05), pages 63-71, XP000982447 ISSN: 0037-9727 cité dans la demande
- PITTENGER M F ET AL: "MULTILINEAGE POTENTIAL OF ADULT HUMAN MESENCHYMAL STEM CELLS" SCIENCE,US,AMERICAN ASSOCIATION FOR THE ADVANCEMENT OF SCIENCE,, vol. 284, no. 5411, 2 avril 1999 (1999-04-02), pages 143-147, XP000867221 ISSN: 0036-8075 cité dans la demande
- HAM R ET AL.: "Improved media for normal human muscle satellite cells : serum-free clonal growth and enhanced growth with low serum" IN VITRO CELL DEV BIOL, vol. 24, no. 8, - août 1988 (1988-08) pages 833-844, XP000982458 cité dans la demande
- HONGPAISAN JARIN: "Inhibition of proliferation of contaminating fibroblasts by D-valine in cultures of smooth muscle cells from human myometrium." CELL BIOLOGY INTERNATIONAL, vol. 24, no. 1, mai 2000 (2000-05), pages 1-7, XP002175030 ISSN: 1065-6995
- SEALE PATRICK ET AL: "A new look at the origin, function, and "stem-cell" status of muscle satellite cells." DEVELOPMENTAL BIOLOGY., vol. 218, no. 2, 15 février 2000 (2000-02-15), pages 115-124, XP002161313 ISSN: 0012-1606
- DATABASE BIOSIS [en ligne] BIOSCIENCES INFORMATION SERVICE, PHILADELPHIA, PA, US; 16 novembre 2000 (2000-11-16) VILQUIN JEAN-THOMAS ET AL: "Clinical report of autologous muscle cell transplantation for treatment of ischemic heart failure." Database accession no. PREV200100316494 XP002175031 & BLOOD, vol. 96, no. 11 Part 1, 16 novembre 2000 (2000-11-16), page 425a 42nd Annual Meeting of the American Society of Hematology;San Francisco, California, USA; December 01-05, 2000 ISSN: 0006-4971
- LEE JOON YUNG ET AL: "Clonal isolation of muscle-derived cells capable of enhancing muscle regeneration and bone healing." JOURNAL OF CELL BIOLOGY, vol. 150, no. 5, 4 septembre 2000 (2000-09-04), pages 1085-1099, XP002161665 ISSN: 0021-9525
- KUÇI ET AL: 'Phenotypic and functional characterization of mobilized peripheral blood CD34+ cells coexpressing different levels of c-Kit' LEUKEMIA RESEARCH vol. 22, 1998, pages 355 - 363
- TAYLOR ET AL: 'Regenerating functional myocardium: improved performance after skeletal myoblast transplantation' NATURE MEDICINE vol. 4, no. 8, Août 1998, pages 929 - 933
- TAYLOR ET AL: 'Delivery of primary autologous skeletal myoblasts into the rabbit heart by coronary infusion: a potential approach to myocardial repair' PROCEEDINGS OF THE ASSOCIATION OF AMERICAN PHYSICIANS vol. 109, no. 3, 1997, pages 245 - 253, XP008106792

## Description

La thérapie cellulaire est une technique à fort potentiel pour le traitement de nombreuses pathologies. Le principe de la thérapie cellulaire repose sur la possibilité de reconstituer un tissu endommagé ou de restaurer une fonction biologique perdue ou altérée au sein d'un tissu, à partir de cellules spécifiques cultivées *ex vivo* et transplantées sur le tissu malade. Un autre intérêt de la thérapie cellulaire est la possibilité d'utiliser les cellules transplantées comme plate-forme de délivrance d'un produit biologiquement actif, si besoin après modification génétique des cellules avant transplantation. De nombreux essais de thérapie cellulaire sont décris à partir de cultures primaires de différents types cellulaires. On peut citer les transplantations de cellules neuronales réalisées pour le traitement de la maladie de Huntington (1) ou la maladie de Parkinson (2), les transplantations de cellules d'ilôts de Langerhans réalisées pour le traitement du diabète (3), ou encore les transplantations de cellules myoblastiques réalisées pour le traitement de la dystrophie musculaire de Duchenne (4, 5, 6, 7) ou après modification génétique des cellules, pour le traitement du nanisme (8), de l'hémophilie (9) et de la maladie de Parkinson (10).

La régénération du muscle squelettique est assurée par les cellules satellites qui sont des cellules myogéniques mononuclées situées sous la lame basale des fibres musculaires. A la suite d'une lésion, ces cellules quittent l'état de quiescence pour entrer dans une phase de prolifération active et prennent le nom de myoblastes. Par la suite, les myoblastes fusionnent pour former les myotubes. Des essais de transplantation de myoblastes ont été réalisés chez l'homme pour traiter la dystrophie musculaire de Duchenne et la dystrophie musculaire de Becker (4, 6, 7, 11). Si l'effet fonctionnel des transplantations décrites dans ces études reste limité, aucun effet secondaire n'a été rapporté en terme d'infection ou de carcinogenèse.

Par ailleurs, l'utilisation de cellules myoblastiques dans le traitement des cardiopathies, et en particulier de l'insuffisance cardiaque post-isçhémique a été envisagée. En effet, contrairement au tissu musculaire, les tissus myocardiques sont dépourvus de cellules souches capables de former des cadiomyocytes et régénérer des tissus. Le traitement le plus radical de l'insuffisance cardiaque post-ischémique reste à l'heure actuelle, la transplantation cardiaque. Cependant, la pénurie de greffons limite cet usage thérapeutique. La transplantation de cellules issues de tissus musculaires dans le muscle cardiaque a donc été envisagée en tant qu'alternative à la transplantation cardiaque. Des études de transplantation de myoblastes dans le tissu infarci du myocarde ont été réalisées chez le rat, le lapin ou le chien (12, 13, 14). Les résultats de ces études ont montré la faisabilité et une certaine efficacité fonctionnelle de telles transplantations. Des essais de greffes de cardiomyocytes foetaux dans un modèle d'insuffisance cardiaque d'origine iatrogène chez la souris ont aussi montré un certain bénéfice fonctionnel. Cependant, l'utilisation de cellules foetales pose, dans la perspective d'application clinique, de nombreux problèmes éthiques, immunologiques et d'approvisionnement cellulaire. L'utilisation d'une population de cellules myoblastiques issues du muscle squelettique constitue donc une alternative particulièrement intéressante pour la préparation de produit de thérapie cellulaire pour le traitement de l'insuffisance cardiaque post-ischémique, voire pour le traitement de cardiopathies d'origines diverses.

L'un des types cellulaires les plus importants contenu dans le tissu musculaire est la cellule satellite, précurseur du myoblaste. C'est le type cellulaire qui a été utilisé dans les différentes études cliniques. Cependant, le tissu musculaire contient d'autres types cellulaires. En particulier, certaines cellules d'origine musculaire pourraient aussi être utilisées pour la reconstitution d'un potentiel hématopoïétique (15, 16). Une étude in vitro a aussi démontré la présence dans le muscle humain de progéniteurs susceptibles de se différencier à long terme en tissus cartilagineux ou osseux (17). Des exemples de milieux permettant la différenciation en tissu adipeux, cartilagineux ou osseux sont décrits pour des cellules souches mésenchymateuses (22).

En conséquence, compte tenu des propriétés de différenciation des cellules d'origine musculaire, l'utilisation de ces cellules en thérapie cellulaire apparaît intéressante pour le traitement de nombreuses lésions affectant les tissus du système hématologique et immunologique, les tissus osseux, adipeux, cartilagineux, musculaires ou vasculaires.

Une des difficultés majeures de la thérapie cellulaire reste l'obtention d'une population de cellules suffisamment grande, homogène et dont le degré de différenciation est adapté à l'effet désiré.

Des procédés de préparation cellulaire myoblastique et leur utilisation en thérapie cellulaire sont décrits dans l'état de la technique (4, 18, 19, 20, 21, 25, 26, 27). La majorité des procédés comprennent :
- une étape de prélèvement des tissus musculaires par biopsie,
- une étape d'éminçage,
- une étape de dissociation des fibres musculaires par action enzymatique,
- une étape de séparation des cellules individualisées par filtration,
- une étape de sélection des cellules myoblastiques par clonage ou par tri cellulaire.

Afin d'obtenir une population suffisamment dense et riche, voire des populations pures en cellules myoblastiques, il a été proposé de sélectionner les cellules myoblastiques sur la base de l'expression de marqueurs spécifiques. Ainsi, la sélection des cellules myoblastiques peut se faire par le clonage des cellules et la caractérisation ultérieure des clones obtenus par cytofluorimétrie puis sélection des cellules musculaires squelettiques exprimant l'antigène CD56 (7). Une méthode directe de tri des cellules myoblastiques exprimant l'antigène CD56 par cytofluorimétrie de flux et ses avantages pour l'obtention d'une culture pure de myoblastes sont aussi décrits dans l'état de la technique (21).

Les cellules conservées sont ensuite cultivées dans un milieu de culture modifié et spécialement adapté pour la culture des myoblastes (23).

La présente invention résulte de l'observation que les cellules issues du tissu musculaire squelettique présentent un potentiel de régénération de nombreux tissus en fonction de leur degré de différenciation. L'invention proposée permet donc de fournir des populations cellulaires bien caractérisées d'origine musculaire.

La présente invention fournit un procédé d'obtention d'une population cellulaire comprenant un type cellulaire dominant, à partir d'une biopsie de tissu musculaire, ledit procédé comprenant les étapes suivantes :
a) prélèvement et éminçage d'une biopsie musculaire ,
b) dissociation enzymatique des fibres et des cellules musculaires et séparation des cellules individualisées par filtration,
c) mise en culture des cellules d'origine musculaire ainsi obtenues dans un réacteur de culture de cellules adhérentes en présence d'un milieu comprenant MCDB 120 ET D-valine substituée à la L-valine suivie le cas échéant d'une ou plusieurs phases d'expansion,
d) identification des types cellulaires présents aux différents stades de la culture par l'analyse de marqueurs cellulaires spécifiques,
e) choix du stade de culture pendant lequel le type cellulaire recherché est en proportion dominante dans la population de cellules,
f) récolte d'une population de cellules au stade de culture choisi en e),
g) le cas échéant, congélation des cellules prélevées à l'étape choisie.

L'étape d) est facultative dans la mesure où, lorsque le procédé est utilisé plusieurs fois dans les mêmes conditions, l'expérimentateur connaît les types cellulaires présents aux différents stades de culture et leurs proportions relatives sans avoir à répéter l'étape d'identification.

Il a en effet été constaté que l'étape d'identification (d) conduisait à des résultats sensiblement identiques lorsque le même procédé était répété.

Dans un mode de réalisation particulier de l'invention, le procédé comprend en plus la mise en oeuvre de techniques de déplétion ou d'enrichissement avant l'étape de culture c) ou avant expansion, afin de modifier les proportions des différents types cellulaires.

Les termes « population cellulaire » ou « population de cellules » signifient toute population de cellules non pures, contenant en général un type cellulaire dominant et un ou plusieurs types cellulaires minoritaires. Le type cellulaire dominant est le type cellulaire dont la proportion dans la population de cellules est la plus élevée. Préférentiellement, un type cellulaire dominant est le type cellulaire dont la proportion dans la population de cellules dépasse 50%. Un produit de thérapie cellulaire apte à l'administration humaine comprend une solution isotonique dans laquelle les cellules sont resuspendues. Cette solution doit être exempte des composants toxiques présents dans les milieux de congélation. Un tel composant est, par exemple, le DMSO.

L'invention résulte en particulier de la constatation que le procédé permet d'obtenir une population de cellules dont la composition est adaptée à l'effet thérapeutique recherché. Il permet d'obtenir une population de cellules dont le type cellulaire dominant exprime le marqueur CD56+ et le marqueur HLA de classe I sans clonage préalable, ni sélection positive des cellules exprimant le marqueur CD56+.

La biopsie musculaire est en général réalisée par prélèvement de cubes de 2 à 4 cm de coté. On peut prélever selon les besoins de 0,05 grammes jusqu'à plusieurs dizaines de grammes. Un des avantages du procédé est qu'il permet d'obtenir un très grand nombre de cellules d'un type cellulaire en proportion dominante, variant de quelques milliers à plusieurs milliards, selon le type cellulaire recherché, le nombre d'expansions réalisées, et le temps alloué pour chaque passage. A titre d'exemple, le procédé permet de produire jusqu'à plusieurs centaines de millions de cellules exprimant l'antigène CD56 dans un délai de deux à trois semaines. Le procédé autorisant de nombreuses phases d'expansion, il permet notamment l'obtention théorique d'au moins 100 milliards de cellules exprimant l'antigène CD56+ et l'antigène HLA de classe I au terme de 8 à 9 expansions. Le tissu musculaire prélevé est un tissu musculaire squelettique, préférentiellement prélevé chez l'adulte, le jeune adulte, l'adolescent ou l'enfant. Dans une forme de réalisation de l'invention, le tissu musculaire prélevé est un tissu musculaire squelettique foetal. Les cellules peuvent être notamment obtenues à partir du vaste externe, vaste interne, biceps, quadriceps, jambiers, gastrocnémiens, péronier, deltoïdes, grand dorsal, sterno-cleido-mastoïdien, intercostal, homo-hyoïdien, grand droit ou du psoas.

L'éminçage consiste à découper la biopsie en sections d'une taille de préférence inférieure à 0,5 mm placées dans un milieu de culture adapté. L'éminçage est une étape essentielle pour permettre une dissociation enzymatique ultérieure efficace. L'éminçage peut être réalisé manuellement à l'aide de ciseaux fins. Cependant, de manière inattendue, il a été constaté que, lorsque l'étape d'éminçage est réalisée de manière assistée, à l'aide par exemple de broyeurs à couteaux mûs par l'énergie électrique ou mécanique, le procédé de l'invention dans lequel le milieu de culture est adapté à la différenciation en myoblaste permet d'obtenir une population dont le pourcentage de cellules exprimant l'antigène CD56 est particulièrement élevé. Un exemple d'un tel broyeur utilisable est le broyeur Medimachine ® (distribué par Becton-Dickinson).

Par conséquent, dans un mode de réalisation, le procédé de l'invention est caractérisé en ce que l'étape d'éminçage est assistée à l'aide de broyeurs à couteaux, mécaniques ou électriques.

Les tissus musculaires sont constitués de fibres musculaires. Les cellules satellites sont situées sous la lame basale des fibres musculaires. L'étape de dissociation des fibres musculaires et de décollement des cellules satellites est donc une étape nécessaire pour leur isolement. L'étape de dissociation consiste en l'utilisation d'enzymes de digestion de la matrice extracellulaire, elle peut être complétée par une dissociation mécanique par aspiration et refoulement de la suspension au travers d'une pipette.

Le choix des enzymes et leurs concentrations utilisées pour la dissociation des fibres musculaires et des cellules satellites de l'éminçat est guidé par l'étude de leur efficacité enzymatique, les critères recherchés sont une concentration d'enzyme la moins élevée possible et un temps d'incubation minimal pour une efficacité similaire. Le rendement en cellules obtenus après filtration dépend en partie de la qualité de l'étape de dissociation enzymatique. Des enzymes de digestion utilisables dans le procédé de l'invention seules ou en association sont par exemple :
- toutes les collagénases, incluant les types IA, S et H partiellement purifiées, ainsi que la forme purifiée commercialisée sous le nom de Libérase par Roche-Boehringer,
- les trypsines, de toutes origines, en solution dans des tampons contenant ou non de l'EDTA,
- les dispases (aussi connues sous le nom de protéases),
- la pronase,
- les élastases,
- ou encore, les hyaluronidases.

De manière préférée, l'étape de dissociation se fait en deux temps ; une première incubation en présence de collagénase et une deuxième incubation en présence de trypsine. Lorsque la libérase est utilisée, des concentrations d'utilisation dans l'éminçat particulièrement efficaces se situent entre 0.05 à 2 mg/ml. Le temps d'incubation à 37°C pour de telles concentrations étant choisi alors dans une gamme de temps s'étalant de 15 minutes à 2 heures. De préférence, l'activité des enzymes de dissociation est neutralisée après dissociation ou décollement de la couche cellulaire dans le but d'éviter l'endommagement des cellules.

Après neutralisation de l'activité enzymatique, par addition, par exemple, de sérum de veau foetal, de sérum humain autologue, ou de sérum humain allologue provenant d'un groupe compatible, ou encore d'un inhibiteur d'activité enzymatique, le produit de digestion est ensuite filtré sur tamis, sous pression de la gravité afin d'éliminer les fibres non dissociées et recueillir les cellules décollées des fibres musculaires. De manière préférée, une étape de filtration sera réalisée en deux temps : Une étape de filtration sur un tamis de 100 µm, puis une seconde étape sur un tamis de 40 µm.

Les cellules recueillies après filtration sont transvasées dans un réacteur de culture en présence d'un milieu dont la composition permet leur croissance ou/et leur différenciation. La composition du milieu est choisie en fonction du type cellulaire dominant souhaité en fin de culture. A ce stade, une partie de la préparation peut être congelée. Cela peut être une partie de la préparation initiale ou une sous-population issue d'une étape d'enrichissement ou de déplétion. Les milieux de culture utilisés contiennent un ou plusieurs facteurs de croissance et/ou de différenciation dont le rôle est de diriger les cellules progénitrices vers une voie de différenciation spécifique et de les faire proliférer. A titre d'exemple de facteurs de croissance, on peut citer les facteurs de croissance des fibroblastes, bFGF, aFGF, FGF6, le facteur de croissance des hépatocytes, HGF/SF, de l'épiderme, EGF et les différents facteurs caractérisés tels IGF-1, PDGF, LIF, VEGF, SCF, TGFb, TNFa, IL-6, IL-15, NGF, la neuréguline, la thrombopoïétine et l'hormone de croissance. On peut les associer à différentes hormones ou molécules actives qui peuvent entrer dans la composition des milieux, telles que les glucocorticoïdes (naturels ou hémi-synthétiques, i.e. l'hydrocortisone, la dexaméthasone, la prednisolone ou la triamcinolone), les progéstagènes et dérivés (progestérone), les oestrogènes et dérivés (oestradiol), les androgène et dérivés (testostérone), les minéralocarticoïdes et dérivés (aldostérone), les hormones LH, LH-RH, FSH et TSH, les hormones thyroïdiennes T3, T4, l'acide rétinoïque et ses dérivés, la calcitonine, les prostaglandines E2 et F2/alpha ou l'hormone parathyroïdienne.

Avant la mise en culture, ou au cours d'une phase d'expansion, la préparation peut faire l'objet d'enrichissement ou de déplétions. Ces opérations sont réalisées par l'homme du métier en utilisant les différentes techniques proposées dans l'état de la technique pour opérer un tri sélectif. Ces techniques reposent sur l'identification d'antigènes extracellulaires caractéristiques de tel ou tel type cellulaire par des réactifs spécifiques. A titre d'exemple, les antigènes CD34 et CD56 exprimés sur certaines cellules présentes au sein de la population de cellules musculaires peuvent être utilisés. Le tri Initial de la population cellulaire totale selon l'expression de ces deux antigènes permet ainsi de séparer deux groupes. En particulier, il permet de séparer les types cellulaires CD34+ des types cellulaires CD34. Il a été montré que la population CD34+ génère un type cellulaire dominant CD15+, CD56- n'exprimant pas la desmine. La population CD34 génère un type cellulaire dominant CD56+, CD15-exprimant la desmine. Dans de rares cas, la population CD34- génère une population CD56-, CD15-, En particulier, le procédé de l'invention comprend une étape de déplétion des cellules CD34- ou CD34+ conduisant respectivement à un population comprenant un type cellulaire dominant C015+ ou CD56+.

Un mode de réalisation du procédé de l'invention porte donc sur un procédé d'obtention d'une population de cellules dont un type cellulaire dominant est le type cellulaire myoblastique caractérisé en ce qu'il comprend une étape de déplétion des cellules CD34+ avant la mise en culture des cellules ou au cours de l'une des phases d'expansion.

Les cellules sont ensuite mises en culture dans un réacteur adapté pour la culture de cellules adhérentes. Afin de s'affranchir des contraintes de contrôles de la vitesse d'agitation, de sa régularité et de l'homogénéité des préparations, le réacteur de culture est de préférence statique. Il doit présenter une grande surface de culture par rapport aux supports classiques (boites de Pétri, flasques) de manière à récolter en quelques jours une population cellulaire importante. Une exemple d'un tel réacteur de culture est le dispositif de culture en plateaux (simple, double et/ou multi-étagé).

Le dispositif de culture utilisé dans le procédé permet de plus l'échantillonnage des cellules de manière stérile afin de réaliser les prélèvements nécessaires à l'identification des types cellulaires présents aux différents stades de la culture par analyse de marqueurs spécifiques. Il permet la vidange des milieux, le lavage et le décollement des cellules et enfin leur récolte de manière stérile.

De manière préférée, des poches sont utilisées et des tubulures stériles spécialement adaptées relient les poches au réacteur pour permettre les transvasements des milieux ou la récolte des cellules. Ce dispositif permet ainsi de réaliser un grand nombre d'opérations en système clos. Selon la population cellulaire souhaitée, le nombre de jours de culture varie de 0 à 45 jours.

De plus, la culture peut être poursuivie par des techniques classiques d'expansion ou de perfusion pendant une durée pouvant atteindre plusieurs mois.

Afin d'augmenter le nombre de cellules récoltées, une ou plusieurs phases d'expansion sont possibles. Les phases d'expansion comprennent une étape de décollement des cellules, de lavage des cellules et de remise en culture sur une plus grande surface de culture, les solutions et enzymes utilisées pour réaliser ces étapes étant bien connues de l'homme du métier. En particulier, dans un mode de réalisation préféré utilisant un milieu de culture approprié pour la différentiation en myoblaste, le procédé de l'invention comprend au moins trois phases d'expansions de cellules. Un tel procédé permet de multiplier le nombre de cellules sans modifier sensiblement les proportions des types cellulaires obtenues en fin de culture de chaque expansion.

Une cinétique de différenciation est réalisée dans le procédé de l'invention par l'identification des types cellulaires présents dans les populations de cellules obtenues aux différents stades de la culture. Dans le texte qui suit, on désignera l'étape d'identification des types cellulaires présents aux différents stades de la culture par le terme « caractérisation des populations cellulaires ». Cette caractérisation est réalisée à partir d'échantillons prélevés lors de la mise en culture, au cours de la culture et lors de la récolte des cellules. La caractérisation des populations cellulaires peut aussi porter sur une culture de cellules réalisée en parallèle à plus petite échelle mais dans des conditions identiques ou équivalentes en terme de milieu de culture et de procédé de réalisation des phases d'expansion. La caractérisation porte sur les cellules adhérentes ou les cellules présentes dans le surnageant. Les différents stades de la culture sont comptés en jours à partir du jour de mise en culture en réacteur jusqu'à l'apparition des premières cellules différenciées ou l'obtention d'un degré de confluence des cellules d'au moins 20 à 50%. La caractérisation des populations de cellules a pour fonction l'identification de l'ensemble des types cellulaires et de leurs proportions en fonction du stade de la culture. Elle permet en particulier de mettre en évidence les types cellulaires dominants en fonction du stade de la culture. Le procédé de l'invention propose ainsi un moyen de choisir une population cellulaire en fonction des caractéristiques des différents types cellulaires présents dans la population.

Cette caractérisation est réalisée par l'analyse de marqueurs cellulaires par cytofluorimétrie de flux ou FACS, après marquage des antigènes de surfaces ou de tout antigène spécifique des différents types cellulaires à analyser. Dans le présent texte, le terme « marqueurs cellulaires » indique tout antigène cellulaire permettant d'apporter des informations à lui seul ou en combinaison avec d'autres marqueurs sur un type cellulaire. Tout type de marqueur cellulaire peut être utilisé par le procédé, le choix des marqueurs utilisés sera guidé par le choix des types cellulaires que l'on souhaite caractériser.

Toute technique apte à caractériser un antigène cellulaire peut être mise en oeuvre dans le procédé de l'invention. On peut citer à titre d'exemple sans être limitatif, le Western Blot ou l'immunocytochimie. Les techniques permettant la caractérisation des ARN messagers codant lesdits antigènes sont utilisables de manière équivalente.

Les marqueurs cellulaires analysés pour l'identification des types cellulaires sont spécifiquement choisis parmi les marqueurs suivants : CD10, CD13, CD15, CD16, CD34, CD38, CD40, CD44, CD45, CD56, CD71, CD80, CD117 ou encore les structures suivantes, CD138, HLA-Classe I, HLA-classe II, VLA3, VLA5, VLA6, ICAM-1, VCAM et la desmine. De manière préférée, les marqueurs CD10, CD13, CD15, CD34, CD44, CD56, CD117 et HLA Classe I et la desmine sont utilisés. Ces marqueurs sont identifiés par l'utilisation d'anticorps monoclonaux spécifiques. Le tableau 1, ci-après fourni dans la partie expérimentale, indique, pour chacun des marqueurs, les types cellulaires exprimant classiquement les antigènes correspondants. Il est à noter que ces marqueurs cellulaires ont été développés initialement pour la caractérisation du système immuno-hématologique. Une originalité de l'invention consiste en l'utilisation de ces marqueurs pour la caractérisation des cellules d'origine musculaire aux différentes étapes de la culture ou de l'expansion.

Le procédé de l'invention est donc mis en oeuvre grâce à l'analyse de marqueurs cellulaires non classiquement utilisés dans la caractérisation des cellules issues de tissus musculaires. Ces marqueurs cellulaires sont par exemple CD10, CD13, CD15, CD34, CD44, CD45, CD117 et HLA Classe I.

Il a été effectivement observé que la mise en oeuvre du procédé de l'invention permet la mise en évidence d'une évolution au cours du temps du type cellulaire dominant présent dans la culture. De manière surprenante, aux stades précoces de la mise en culture des cellules de tissus musculaires et dans un milieu adapté pour la différenciation myoblastique, les populations majoritaires sont celles exprimant les marqueurs de cellules progénitrices de la moelle et des cellules du système lymphoblastoïde. Plus précisément, il a été observé à J0 et J1, une majorité de cellules de phénotype CD34+/45- ainsi que la présence d'un type cellulaire minoritaire CD117+ dans la population de cellules non adhérentes. L'évolution est ensuite marquée par une augmentation croissante de la proportion de cellules CD15+ et/ou CD56+, et une diminution des populations CD34+. Néanmoins, le type cellulaire dominant reste CD34+ de J0 à J5. Une transition progressive d'une population de type cellulaire dominant CD34+ à CD56+ est observée. Par ailleurs, la proportion des types cellulaires CD13+, CD44+, CD71+ augmente avec le temps. Des populations minoritaires CD138+, HLA-Classe II+ et CD38+ sont aussi observées. Au terme de la culture, le type cellulaire dominant est de phénotype CD56+, en particulier à partir du stade J5 jusqu'à la fin de la culture. Les cellules CD56+ expriment également CD10, CD13, CD44, la desmine, HLA-Classe I, CD44, VLA-3, VLA-5 et VLA-6. Ces marqueurs caractérisent les cellules myoblastiques. Un deuxième type cellulaire prépondérant est constitué de cellules exprimant CD15, CD10, CD13 et HLA-Classe I. Une population CD56-/CD15-/CD13+ est aussi présente en proportions variables constituant un troisième type cellulaire prépondérant. Dans cette population, une partie exprime la desmine et une partie ne l'exprime pas. La mise en oeuvre du procédé de l'invention a ainsi permis de distinguer trois types cellulaires présents dans les populations de cellules, dont les proportions respectives évoluent de manière importante au cours de la culture, les cellules CD34+, les cellules CD15+ et les cellules CD56+.

Dans un mode de mise en oeuvre préféré du procédé de l'invention, la caractérisation des populations cellulaires consiste à déterminer le pourcentage respectif des trois types cellulaires CD34+, CD15+ et CD56+ aux différentes étapes de la culture.

Dans une forme de réalisation de l'invention, un type cellulaire recherché dans une population cellulaire est séparé après sélection du stade de culture pendant lequel le type cellulaire recherché est dans l'état qualitatif ou quantitatif recherché, en particulier, après sélection du stade de culture pendant lequel le type cellulaire recherché est dominant. La présence en proportion dominante des cellules à séparer facilite leur préparation. L'invention fournit ainsi un procédé d'obtention d'une population cellulaire de degré de pureté élevé. On entend par population cellulaire de degré de pureté élevé, une population de cellules dans laquelle le type cellulaire dominant est en proportion supérieure à 50% dans la population cellulaire. Il apparaît clairement que l'homme du métier pourra mettre en oeuvre les différentes techniques proposées dans l'état de l'art pour faire un tri sélectif des dites cellules. A titre d'exemple, citons les techniques de tri par clonage, par cytofluorimétrie de flux ou encore par colonnes d'immunoaffinité ou immunomagnétiques utilisant des anticorps spécifiques des cellules en cause.

Dans un autre mode de réalisation, au contraire, le procédé de l'invention est caractérisé en ce qu'il ne comprend pas d'étapes de purification, de sélection positive, ou de clonage d'un type cellulaire spécifique. Par sélection positive, il faut comprendre toute étape permettant de trier les cellules sur la base d'au moins une caractéristique particulière et notamment l'expression d'un marqueur cellulaire. Il a été montré en particulier, que le procédé de l'invention, contrairement aux procédés décrits dans l'état de la technique, permet d'obtenir un produit de thérapie cellulaire comprenant un type cellulaire dominant de type myoblaste, sans étape de sélection préférentielle du type cellulaire myoblastique. L'absence d'étapes de clonage, de purification ou de sélection positive, permet d'améliorer nettement le rendement final obtenu au terme des procédures d'expansion en terme de nombres de cellules obtenues.

Dans le procédé de l'invention, après arrêt de la culture au stade de culture choisi, un aliquot de cellules peut être prélevé et remis en culture séparément. Le milieu peut être le même ou de composition différente. Les milieux de culture sont choisis en fonction de la voie de différenciation souhaitée. Un exemple de milieu permettant la différenciation en myoblastes, en cellules endothéliales, en cellules musculaires lisses ou en myofibroblastes est le milieu MCDB 120 supplémenté par du sérum de veau foetal (23) qui comprend en particulier un glucocorticoïde tel que la dexaméthasone et le bFGF. Un autre exemple de milieu préféré est le milieu MCDB 120 modifié en substitutant la L-valine par la D-valine. Il a en effet été constaté qu'une telle modification permet d'obtenir un milieu particulièrement sélectif pour la différenciation des cellules en myoblastes. Un tel milieu permet d'obtenir, avec le procédé de l'invention une population comprenant un très grand nombre de cellules dont une majorité est de type CD56+ ou HLA Classe 1.

Un milieu de différenciation en tissus adipeux contient en particulier la dexaméthasone, l'isobutyl-methylxanthine, et le cas échéant, du sérum de veau foetal, l'indométhacine et l'insuline. Le maintien de la différenciation est obtenu dans un milieu contenant 10% de sérum de veau foetal et l'insuline. Pour obtenir une différenciation en tissus cartilagineux, les cellules sont centrifugées en micromasses et cultivées dans du milieu sans sérum mais contenant du TGF-béta3. Un milieu de différenciation en tissus osseux contient de la dexaméthasone, du béta-glycérolphosphate, de l'ascorbate, et le cas échéant, du sérum de veau foetal.

La récolte des cellules s'effectue au stade de culture choisi en fonction de la population cellulaire dont on souhaite l'obtention. Les cellules non adhérentes présentes dans le surnageant ou/et les cellules adhérentes peuvent être récoltées par ce procédé. Les populations de cellules présentes dans le surnageant et de cellules adhérentes peuvent être de composition différente. Par conséquent, le mode de récolte des cellules sera fonction de la population cellulaire recherchée. La récolte des cellules adhérentes s'effectue par dissociation enzymatique des cellules et décollement de la couche cellulaire par des techniques connues de l'homme de métier. La récolte des cellules non adhérentes se fait par aspiration.

La mise en oeuvre du procédé ci-dessus incluant l'établissement d'une cinétique de différenciation à partir de cellules issues de biopsies de tissus musculaires permet l'obtention de populations cellulaires caractérisées.

Les populations de cellules peuvent être obtenues par un procédé similaire au procédé d'obtention de populations de cellules décrit plus haut, mais ne comprenant pas d'étape d'identification des types cellulaires aux différents stades cellulaires. En effet, l'étape d'identification des types cellulaires est nécessaire pour le choix du stade de récolte. Dès lors que, ayant mis en oeuvre au moins une fois le procédé de l'invention, l'homme du métier connaît le stade optimal de récolte des cellules en fonction de la population recherchée, celui-ci peut obtenir les mêmes types de populations en limitant le nombre de marqueurs utilisés pour la caractérisation des populations de cellules et les stades au cours desquels il effectue cette caractérisation. Ainsi, par « populations de cellules susceptibles d'être obtenues », il faut donc inclure une population de cellules obtenue par le procédé de l'invention ne comprenant pas nécessairement une étape de caractérisation des populations de cellules au sens de l'invention, telle que décrite plus haut. Le stade de récolte optimal étant connu par une mise en oeuvre d'une cinétique de différenciation sur une culture préalable, effectuée dans les mêmes conditions.

Les différentes populations cellulaires susceptibles d'être obtenues par le procédé de l'invention, avec ou sans étape d'identification sont de différents types selon le stade de culture choisi.

En particulier, aux stades précoces de culture des cellules, l'invention fournit une population cellulaire dont le type cellulaire dominant exprime le marqueur CD34. Lorsque seules les cellules non-adhérentes sont récoltées au stade précoce de culture, la population cellulaire comprend aussi un type cellulaire minoritaire de phénotype CD117+.

Par la mise en oeuvre du procédé décrit ci-dessus dans lequel le milieu de culture est adapté pour la différenciation myoblastique, l'invention fournit une population cellulaire dont le type cellulaire dominant exprime le marqueur CD15.

Enfin, par la mise en oeuvre du procédé décrit ci-dessus et en utilisant un milieu de culture adapté pour la différenciation myoblastique, une forme de réalisation préférée de l'invention fournit une population cellulaire dont le type cellulaire dominant est constitué de cellules myoblastiques. Les cellules myoblastiques sont caractérisées par l'analyse de l'expression du marqueur CD56. Elles sont caractérisées de manière préférée par l'analyse de l'expression du marqueur CD56 en combinaison avec l'analyse des marqueurs CD10, CD13, CD44, HLA Classe I et la desmine. L'analyse de l'expression de' la desmine, protéine intracellulaire du cytosquelette spécifique des cellules myoblastiques et des cellules musculaires différenciées nécessite un protocole adapté de marquage et d'analyse par FACS, détaillé dans la partie expérimentale. La population cellulaire comprend en plus une population doublement négative CD56⁻ /CD15⁻.

L'invention fournit en particulier une population cellulaire issue d'une même biopsie musculaire et comprenant de 50 x 10⁶ à 800 x 10⁹ cellules, de préférence au moins 500 x 10⁶ cellules dont au moins 50% des cellules, et mieux au moins 60%, et de manière encore préférée, au moins 70%, sont CD56+.

L'invention porte sur l'utilisation d'une population de cellules CD56⁺ obtenue selon les procédés décrits plus haut dans la préparation d'un produit de thérapie cellulaire pour le traitement chez l'homme de l'insuffisance cardiaque post-ischémique ou pour la réparation des tissus cardiaques. En effet, la mise en oeuvre du procédé permet l'obtention d'un grand nombre de cellules rapidement et les populations cellulaires obtenues ont l'avantage d'être homogènes, et donc particulièrement adaptées pour la préparation d'un produit de thérapie cellulaire.

Elle porte aussi sur l'utilisation d'une population de cellules issues d'une même biopsie musculaire et comprenant de 500x10⁶ à 800 x 10⁹ cellules dont au moins 50%, et mieux au moins 60%, sont CD56+ ou HLA Classe 1 dans la préparation d'un produit de thérapie cellulaire pour le traitement chez l'homme, de l'insuffisance cardiaque post-ischémique ou toutes cardiopathies d'origine génétique, virale, médicamenteuse, infectieuse ou parasitaire

Le traitement des cardiopathies consiste en particulier à injecter à l'aide d'une aiguille une population de cellules, dont le type dominant a les caractéristiques de cellules myoblastiques, obtenue et préparée comme produit de thérapie cellulaire, directement dans le tissu myocardique (12) ou indirectement dans la circulation artérielle (24).

De préférence, au moins 600 x 10⁶ cellules issues d'une même biopsie sont injectées.

L'insuffisance cardiaque est aujourd'hui prise en charge par traitement par des inhibiteurs de l'enzyme de conversion de l'angiotensine (ACEI). Les expériences décrites ci-après indiquent un effet améliorant certain de la transplantation de cellules myoblastiques in situ dans la zone infarcie lorsque cette transplantation est réalisée concomitamment au traitement par l'ACEI.

L'invention porte donc également sur l'utilisation de populations cellulaires d'origine musculaire obtenues selon les procédés décrits plus haut comme transplant pour potentialiser les traitements pharmacologiques des insuffisances cardiaques.

La partie expérimentale du présent texte décrit la réalisation de transplantations de cellules d'origine musculaire autologues chez le rat démontrant la faisabilité de cette technique. En effet, les résultats montrent que la transplantation de ces cellules chez le rat améliore significativement les paramètres d'évaluation fonctionnelle démontrant par là la faisabilité de telles transplantations. Ils montrent aussi que l'amélioration de la fonction myocardique est liée au nombre de cellules greffées.

La partie expérimentale décrit également les résultats obtenus chez le rat, lors de l'utilisation conjointe de cellules musculaires autologues et du traitement pharmacologique de l'insuffisance cardiaque.

Au cours des phases de culture et d'expansion des cellules dans les procédés fournis par l'invention, une étape de modification génétique des cellules par transfection d'un acide nucléique hétérologue peut être réalisée. L'acide nucléique est choisi de manière à permettre l'expression d'un polypeptide ou d'une protéine dans les cellules transfectées. Les cellules transfectées sont ensuite transplantées et permettent la délivrance du polypeptide ou de la protéine exprimés à partir de l'acide nucléique hétérologue, le dit polypeptide ou protéine étant un produit biologiquement actif. L'invention porte ainsi sur l'utilisation d'une population de cellules comme produit de thérapie cellulaire en tant que plate-forme de délivrance d'un produit biologiquement actif.

La première partie présente les exemples de mise en oeuvre du procédé permettant selon le stade de culture choisi l'obtention de populations cellulaires dont le type cellulaire dominant est CD34+ ou CD15+ ou CD56+ (myoblastique) ou doublement négatif CD56-/CD15-.

Les résultats présentés en deuxième partie montrent l'efficacité de la technique de transplantation de cellules myoblastiques sur des tissus cardiaques infarcis chez le rat. Elle permet aussi de déterminer les critères requis pour une bonne efficacité.

Enfin, une troisième partie présente les essais cliniques réalisés chez l'homme de transplantation de cellules d'origine musculaire pour la reconstitution de tissus myocardiques, la réparation de tissus myocardiques, la génération de tissu métaboliquement actif, la génération de tissu présentant une activité fonctionnelle n'existant pas avant reconstitution. Elle montre en outre que les cellules musculaires peuvent également jouer un rôle dans le remodelage du tissu cardiaque chez l'homme.

### PARTIE EXPERIMENTALE

### Légende des figures:

- Figure 1 :: Graphe indiquant les valeurs de LVEF entre le groupe traité et le groupe contrôle.
- Figure 2 :: Graphe indiquant les valeurs de LVEDV entre le groupe traité et le groupe contrôle.
- Figures 3A et 3B :: Graphe indiquant les valeurs de LVEF à 1 mois (3A) et LVEF à 2 mois (3B) selon les catégories de risque.
- Figure 4 :: Régression linéaire présentant la corrélation entre l'amélioration fonctionnelle et le nombre de cellules injectées.
- Figure 5 :: Echocardiographies pré-opératoire (A) et post- opératoire (B) : On voit nettement l'épaississement systolique de la paroi postérieure initialement akimétique.
- Figure 6 :: Vue horizontale des deux ventricules par imagerie par tomographie à émission de positron présentant la paroi postérieure du ventricule gauche (zone greffée en bas du scan). Les photos 6A et 6B (haut) montrent une activité métabolique homogène dans les parois septales et antérieures avec une diminution de l'activité métabolique dans la paroi postérieure avant l'opération. Les photos 6C et CD (bas) montrent l'assimilation du désoxyglucose ₂fluoro¹⁸ dans la paroi postérieure après opération.
- Figure 7 :: Stabilité des caractéristiques des populations cellulaires (CD56+) en fonction des expansions successives

### A. PROCEDE D'OBTENTION DE POPULATIONS CELLULAIRES ISSUES DE TISSUS MUSCULAIRES SQUELETTIQUES

### A.1 Matériels, solutions et milieux utilisés

Milieu A : Milieu MCDB120 (23) modifié : substitution de la L-Valine par de la D-Valine, élimination du rouge de phénol, élimination de la thymidine.

Milieu B : Milieu A + 20% de sérum de veau foetal irradié + antibiotiques (à 100 Ul/ ml pour la pénicilline et 100 µg/ml pour la streptomycine).

Milieu C : Milieu B + bFGF (10 ng/ml) + dexamethasone (1 µM).

Solution D : PBS

Milieu E : Milieu A + bFGF (10 ng/ml) + dexamethasone (1 µM) + sérumalbumine humaine stérile (0.5%).

Solution F : Solution saline isotonique stérile 0.9% NaCl.

Solution G : Solution F + 4% de sérumalbumine humaine et 7.5% de DMSO (diméthyl-sulfoxyde) final.

Solution H : Solution d'injection F + 0,5% de sérumalbumine humaine

Le procédé d'obtention de populations cellulaires décrit ci-après comporte 6 étapes :
■ prélèvement et éminçage de la biopsie
■ dissociation enzymatique et séparation des cellules indivualisées par filtration
■ mise en culture des cellules et phases d'expansion des cultures
■ identification des types cellulaires présents aux différents stades de la culture par l'analyse de marqueurs cellulaires spécifiques
■ récolte des cellules
■ préparation et/ou maintien en survie et/ou congélation des cellules
■ préparation du produit de thérapie cellulaire

Dans le procédé, la centrifugation des cellules s'effectue à 160 g pendant 5 minutes. Le comptage des cellules et l'analyse des populations sont réalisés à l'aide d'un hémacytomètre de Neubauer. Dans les phases d'expansion, les cellules sont incubées à 37°C dans un incubateur air-CO₂ (95%-5%) saturé en humidité. L'observation des cellules se fait à l'aide d'un microscope inversé à contraste de phase.

### A.2 Résultats

### A.2.1 Prélèvement et éminçage de la biopsie

Le prélèvement est réalisé au bloc opératoire, en milieu stérile, en système ouvert. Une biopsie d'environ 10-16 grammes de tissu musculaire squelettique est réalisée par l'équipe chirurgicale. La biopsie est ensuite découpée en petits cubes de 2 à 4 mm de côté, puis émincée à l'aide de ciseaux fins dans le milieu A. L'éminçat est placé dans une bouteille stérile contenant 25 ml de milieu A.

L'étape d'éminçage est aussi réalisé de manière assistée, à l'aide de broyeurs à couteaux Medimachine ® (distribué par Becton-Dickinson). Dans ce dispositif, des fragments de masse inférieure à 0,2g sont dissociés dans les réceptacles Medicon stériles, à l'issue de broyage contrôlé par un moteur électrique, d'une durée inférieure à 5 minutes. La répétition de l'opération à l'aide de plusieurs réceptacles Medicon permet de préparer finalement plusieurs grammes de muscle. Le tableau A ci-après présente les proportions des types cellulaires CD56+, CD15+ et CD34+ obtenues au cours des différents stades de culture J0, J15, J20 et J26 (la proportion de cellules CD34 étant quasi-nulle à J15, celle-ci n'est pas indiquée dans le tableau pour les étapes suivant la mise en culture J0). On observe que, de manière inattendue, l'étape d'éminçage de la biopsie constitue une étape cruciale pour obtenir rapidement une population comprenant un type cellulaire dominant de type myoblaste CD56+.

**TABLEAU A :**

| COMPARAISON EMINCAGE AUX CISEAUX / BROYAT MECANIQUE | | | | | |
|---|---|---|---|---|---|
| | | | | | |
| | | BIOPSIE 1 | | BIOPSIE 2 | |
| | | EMINÇAGE AUX CISEAUX | BROYAT MECANIQUE | EMINÇAGE AUX CISEAUX | BROYAT MECANIQUE |
| J0 | POIDS | 0,8 g | 0,8 g | 1,2 g | 1,4 g |
| | CNT 10*5 | 2,6 | 2,4 | 6,4 | 4,8 |
| | %CD34+ | 7,4 | 4,7 | 2,6 | 1 |
| | %CD56+ | 0,4 | 0 | 1,2 | 0,4 |
| | %CD15+ | 0 | 0 | 0,9 | 3,3 |
| PASSAGE 1 | JOUR | J15 | J15 | J7 | J15 |
| | CNT 10*5 | 11,7 | 18,4 | 16,3 | 16,3 |
| | %CD56+ | 51,6 | 90,5 | 35,1 | 77,9 |
| | %CD15+ | 1,8 | 0,9 | 44,1 | 2,6 |
| PASSAGE 2 | JOUR | J20 | J20 | J21 | J21 |
| | %CD56+ | 58 | 93,4 | 15 | 89,6 |
| | %CD15+ | 20,9 | 4,4 | 20 | 7,3 |
| PASSAGE 3 | JOUR | J26 | J26 | J27 | J27 |
| | %CD56+ | 43,6 | 75,8 | 49,7 | 60,2 |
| | %CD15+ | 30,1 | 1,7 | 10,9 | 1,1 |

Différentes tailles de prélèvement ont été testées pour la mise en oeuvre du procédé de l'invention, couvrant une gamme de poids de 0,13 g à 14,9 g. Les résultats présentés dans les tableaux suivants montrent que l'évolution des proportions des différents types de population et l'amplification du nombre de cellules évoluent de manière comparable pour des tailles de biopsies de moins de 1 g (tableau B) à plus de 10 g (tableau C).

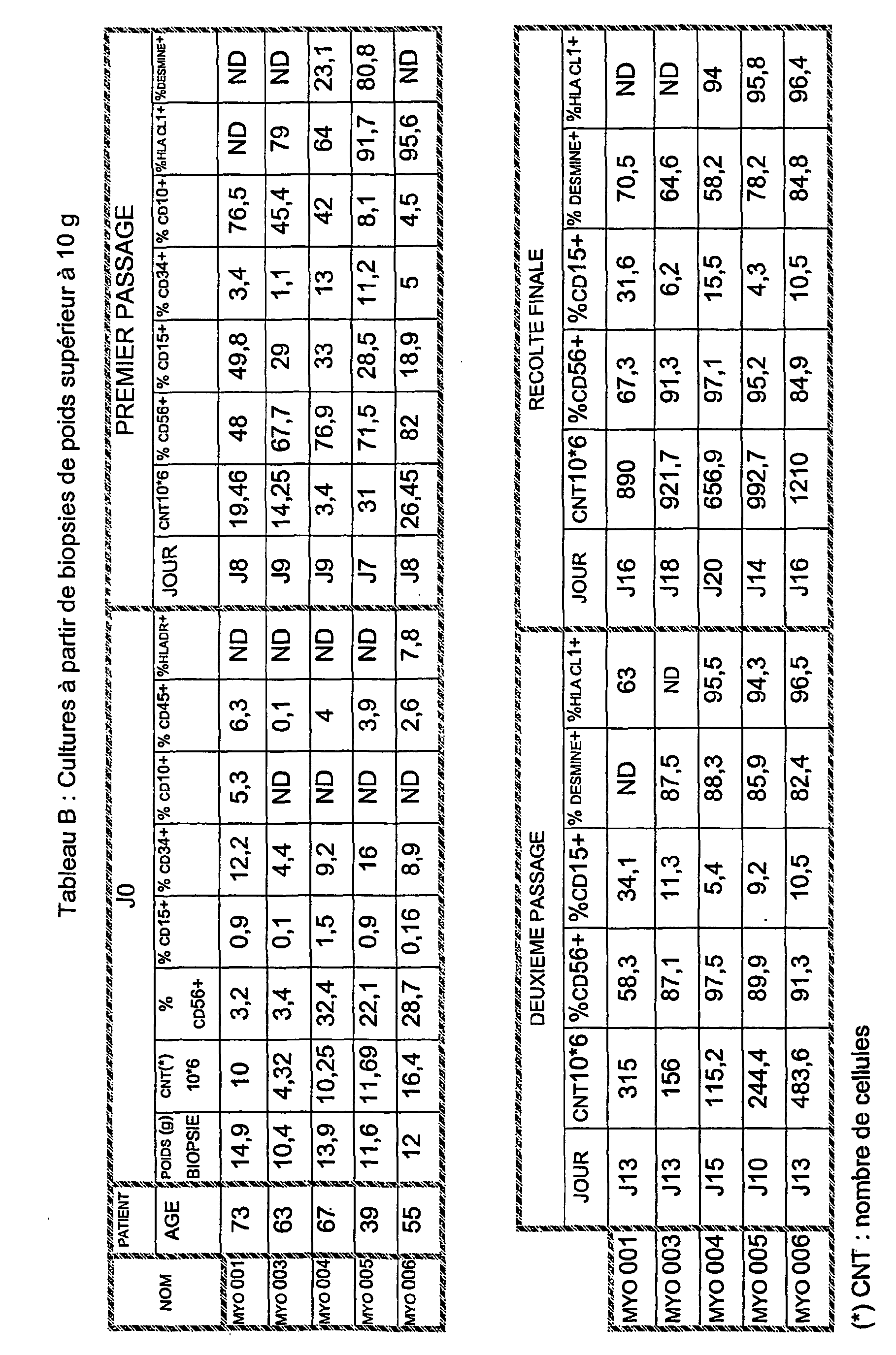

**Tableau C : Cultures à partir de biopsies de poids inférieur à 0,5 g**

| | | | **PASSAGE 1** | **PASSAGE 2** | **PASSAGE 3** | | | | | **PASSAGE 1** | **PASSAGE 2** | **PASSAGE 3** |
|---|---|---|---|---|---|---|---|---|---|---|---|---|
| BIOPSIE | J TRAITEMENT | J0 | J10 | J14 | J16 | | BIOPSIE | J TRAITEMENT | J0 | J6 | J11 | J17 |
| 5007 | CNT 10*5 | 1,2 | 11,2 | 64 | 266 | | 5054 | CNT 10*5 | 2,8 | 6,6 | 7,1 | 370 |
| | % CD34+ | 28,8 | ND | ND | 0 | | | % CD34+ | 10,7 | ND | ND | ND |
| 0,23 g | %CD56+ | 1,95 | 68,6 | 87,6 | 89,5 | | 0,45 g | %CD56+ | 25,7 | 62,3 | 69,1 | 84,6 |
| | %CD15+ | ND | 30,9 | 17,3 | 28,8 | | | %CD15+ | ND | 31 | 26,9 | 14,5 |
| | % VIABILITE | 88,9 | 96 | 100 | 98,7 | | | % VIABILITE | 88,7 | 100 | 96 | 87 |
| BIOPSIE | J TRAITEMENT | J0 | J7 | J10 | J15 | | BIOPSIE | J TRAITEMENT | J0 | J7 | J8 | J15 |
| 5008 | CNT 10*5 | 2,04 | 7 | 21,6 | 586,6 | | 5058 | CNT 10*5 | 7,7 | 9,4 | 13,6 | 534 |
| | % CD34+ | 34 | ND | ND | 0 | | | % CD34+ | 44,3 | 21,4 | ND | ND |
| 0,23 g | %CD56+ | 6,1 | 47,1 | 66,3 | 92,9 | | 0,19 g | %CD56+ | 8,4 | 23,2 | 27,5 | 72,2 |
| | %CD15+ | ND | 44,6 | 30,1 | 9,5 | | | %CD15+ | ND | 63,2 | 63,6 | 18,9 |
| | % VIABILITE | 85,1 | 92 | 88 | 97,9 | | | % VIABILITE | 95 | 82,1 | 100 | 97 |
| BIOPSIE | J TRAITEMENT | J0 | J7 | J10 | J14 | | BIOPSIE | J TRAITEMENT | J0 | J6 | J13 | J15 |
| 5011 | CNT 10*5 | 2,1 | 9,8 | 35 | 285 | | 5060 | CNT 10*5 | 5,2 | 9 | 18,8 | 663 |
| | % CD34+ | 26,2 | ND | ND | ND | | | % CD34+ | 48,7 | ND | ND | ND |
| 0,2 g | %CD56+ | 3,5 | 24,1 | 29,9 | 38 | | 0,33 g | %CD56+ | 7,9 | 42,3 | 52,8 | 89,7 |
| | %CD15+ | ND | 70,8 | 63,8 | 60,5 | | | %CD15+ | ND | 46,8 | 48,6 | 11,9 |
| | % VIABILITE | 85,5 | 98 | 98 | 99 | | | % VIABILITE | 94,4 | 100 | 99 | 98 |

Des biopsies ont été prélevées de patients âgés de 15 à 73 ans. Les résultats présentés dans le tableau D suivant montrent que le procédé est applicable quel que soit l'âge du patient sur lequel la biopsie est prélevée.

**Tableau D : Préparation de cellules d'origine musculaire à partir de biopsies provenant de patients de différents âges**

| | Jour 0 | | | | Premier passage | | Second passage | | Troisième passage | |
|---|---|---|---|---|---|---|---|---|---|---|
| Nom | Poids (g) | Age patient (années) | Nombre 10*5 | % CD56+ | Nombre 10*6 | % CD56+ | Nombre 10*6 | % CD56+ | Nombre 10*6 | % CD56+ |
| MYO1 | 14.9 | 73 | 100 | 3.2 | 19.5 | 48 | 315 | 58.3 | 890 | 67.3 |
| MYO3 | 10.4 | 63 | 43 | 3.4 | 14.2 | 67.7 | 156 | 87.1 | 922 | 91.3 |
| MYO4 | 13.9 | 67 | 102 | 32.3 | 3.4 | 76.9 | 115 | 97.5 | 657 | 97.1 |
| MYO5 | 11.6 | 39 | 117 | 22.1 | 31 | 71.5 | 244 | 89.9 | 993 | 95.2 |
| MYO6 | 12 | 55 | 164 | 28.7 | 26.5 | 82 | 483 | 91.3 | 1210 | 84.9 |
| 4929 | 0.19 | 15 | 1 | ND | 1.6 | 64.3 | 4.7 | 75.4 | 56 | 82.4 |
| 5008 | 0.24 | 45 | 2 | 6.1 | 0.7 | 47.1 | 2.2 | 66.3 | 59 | 92.9 |
| MOS | 3.6 | 84 | 110 | ND | 75 | 93.4 | 240 | 96.4 | 565 | 93.7 |
| CEL | 3.0 | 51 | 45 | ND | 42 | 51.8 | 120 | 63.7 | 548 | 68.7 |

Les biopsies peuvent être conservées 90 h à 4°C ou congelées dans une solution saline équilibrée avant leur mise en culture. Les tableaux E et F suivants montrent que la viabilité des cellules et l'évolution des proportions des différents types de population né sont pas significativement affectées après 90 h de conservation de la biopsie à 4°C, ou après congélation

**Tableau E : Mise en culture d'une biopsie conservée 90 h à +4°C dans une solution saline équilibrée et préparation des cellules musculaires selon le procédé**

| POIDS : 1,05 g | J0 | PASSAGE 1 (J7) |
|---|---|---|
| CNT 10*6 | 0,8 | 4,9 |
| % CD34+ | 7,7 | NS |
| %CD56+ | 6,5 | 58,7 |
| %CD15+ | 8,4 | 37,7 |
| % VIABILITE | 90,5 | 95 |

| | | |
|---|---|---|
| NS : non significatif | | |

**Tableau F : Culture à partir d'une biopsie décongelée**

| | | | PASSAGE 1 | PASSAGE 2 | PASSAGE 3 |
|---|---|---|---|---|---|
| BIOPSIE | J TRAITEMENT | J0 | J18 | J20 | J25 |
| 4929 | CNT 10*5 | 0,97 | 16,2 | 47,3 | 55?7 |
| décongelée | % CD34+ | 20,5 | ND | 0,04 | 0 |
| muscle non | % CD56+ | 44,9 | 64,3 | 75,4 | 82,4 |
| pathologique | % CD15+ | ND | 27,1 | 24,5 | 24,5 |
| POIDS : 0,19 g | % VIABILITE | 90,8 | 100 | 96,8 | 96,7 |

Le procédé de culture peut être mis en oeuvre à partir de biopsies de sujets sains ou de patients atteints de pathologie. Les résultats suivants présentés dans le tableau G montrent en particulier que le procédé de l'invention peut être mis en oeuvre pour la préparation de cellules d'origine musculaire issues d'un patient atteint de la dystrophie musculaire de Duchenne.

**Tableau G : Culture à partir d'une biopsie décongelée provenant d'un patient atteint de la dystrophie musculaire de Duchenne**

| | | | **PASSAGE 1** | **PASSAGE 2** | **PASSAGE 3** | **PASSAGE 4** | **PASSAGE 5** |
|---|---|---|---|---|---|---|---|
| BIOPSIE | STADE | J0 | J7 | J14 | J21 | J26 | J29 |
| 4964 | CNT 10*5 | 1,58 | 1,78 | 11,6 | 473,6 | 2411,2 | 6397 |
| DE 24 H | % CD34+ | 53,9 | ND | 0,3 | 0 | ND | 0 |
| muscle strié | % CD56+ | 5 | 24,6 | 78,1 | 73,7 | 60 | 52,5 |
| paravertébral | % CD15+ | 0,4 | 30,4 | 12,7 | 15,5 | 26,4 | 36,1 |
| POIDS : 0,136 g | % VIABILITE | ND | 94 | 82,5 | 91,2 | 95,6 | 98 |

Les résultats ci-après présentés montrent que le procédé peut être mis en oeuvre à partir de tout type de biopsies musculaires. En particulier, des biopsies ont été obtenues de différents muscles, le paravertébral, le jambier antérieur, le long péronier, l'extenseur commun orteils, le long péronier latéral, le jambier postérieur et le soléaire. Les résultats obtenus en fonction de différentes biopsies sont présentés dans le tableau H.

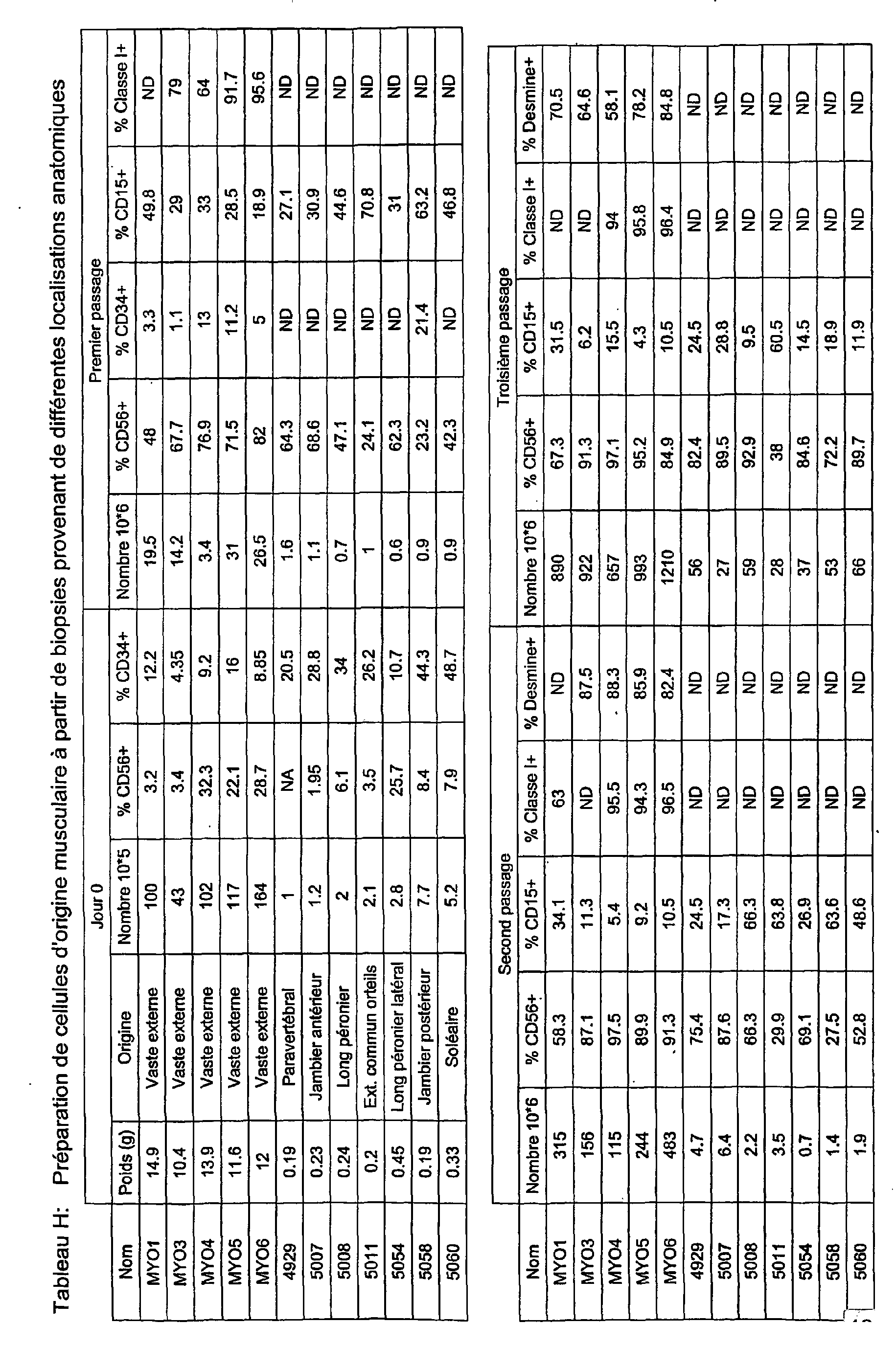

### A.2.2 Dissociation enzymatique et séparation des cellules individualisées par filtration

Le flacon contenant l'éminçat est centrifugé à température ambiante. Le surnageant est éliminé par aspiration. Le poids de l'éminçat est obtenu par pesée sur une balance tarée à l'aide d'un flacon vide. L'éminçat est rincé avec 25 ml du milieu A. Après sédimentation des éminçats, le surnageant est éliminé par aspiration.

Une solution de libérase (Roche-Boehringer) a été préparée selon les instructions du fabricant puis reconditionnée et congelée à la concentration de 10 mg/ml. La libérase est décongélée extemporanément puis ajoutée à l'éminçat à concentration de 0.1 mg/ml, sous un volume de 10 mL par gramme de tissu. La bouteille est placée à l'étuve à 37°C pour une durée de 60 minutes. La bouteille est agitée manuellement toutes les 5 à 10 minutes (diffusion de l'enzyme et dissociation mécanique douce). La suspension est ensuite centrifugée. Le surnageant est éliminé par aspiration à la pipette. Ce premier produit de digestion est ensuite incubé dans une solution de trypsine à 0.25%. On utilise 10 ml de solution enzymatique par gramme de tissu initial. La suspension est digérée durant 20 minutes à 37°C à l'étuve, avec agitation manuelle toutes les 5 minutes. Elle est ensuite aspirée et refoulée au travers d'une pipette de 25 ml. 10% de sérum de veau foetal irradié (Hyclone) est alors ajouté pour la neutralisation des activités enzymatiques.

Le produit de digestion est filtré sur tamis de 100 µm, puis de 40 µm, sous pression de la gravité, afin de séparer les cellules dissociées des tissus résiduels. Un tamis par gramme de tissu est utilisé (Cell strainer de Falcon). Le filtrat est centrifugé à 300 g durant 5 minutes. Après élimination du surnageant, les culots sont lavés à l'aide du milieu B, puis centrifugés. Le surnageant est éliminé par aspiration. Le culot est ensuite repris dans 10 ml de milieu C. 100 µl de volume est prélevé pour comptage. Une aliquote est réservée pour estimation de la viabilité par cytofluorimétrie (iodure de propidium).

### A.2.3 Mise en culture des cellules et phases d'expansion des cultures

Après déconditionnement, les cellules sont transvasées dans le dispositif de culture. Le dispositif de culture est un plateau de culture (Nunc Single Tray) d'une surface de 600 cm². Le remplissage du plateau se fait par un orifice prévu à cet effet et obturé par un bouchon stérile à usage unique. Les cultures sont incubées à 37°C dans une enceinte saturée en humidité en atmosphère contrôlée air-CO₂ (95%-5%).

Le lendemain de la mise en culture, une première vidange est réalisée afin d'éliminer les cellules mortes et les débris musculaires. Une poche vide est connectée à l'un des deux orifices du dispositif de culture. Le milieu est éliminé par gravité et remplacé par 120 ml de milieu C qui sont ajoutés à la culture. Le milieu C est renouvelé au bout de 120 à 192 heures. L'expansion est décidée lorsque le degré de confluence des cellules atteint 20% à 50% ou lorsque les premiers myotubes apparaissent (environ 8 jours après la mise en culture).

Après vidange du milieu, les cellules sont lavées par agitation manuelle douce à l'aide de 50 ml de solution D. La solution D est vidangée puis 20 ml de solution de trypsine irradiée (0.25%) sont ajoutés. Le flacon est mis à incuber 5 minutes à 37°C. Les cellules sont récoltées dans une poche de 40 ml. L'action de la trypsine est neutralisée par l'addition de 10% de sérum de veau foetal. Le sérum est injecté à l'intérieur de la poche à l'aide d'une seringue. Les cellules sont centrifugées. Le surnageant est éliminé par transvasement dans une autre poche de vidange, reliée par un connecteur stérile. Le culot est remis en suspension dans 30 ml de milieu C pour lavage des cellules puis les cellules sont centrifugées. Le surnageant est éliminé par transvasement dans une autre poche de vidange, reliée par un connecteur stérile. Le culot cellulaire est resuspendu dans 20 ml de milieu C. Un aliquot est prélevé pour numération et analyse des populations. La viabilité est estimée à l'aide d'un cytofluorimètre. Les cellules sont ensuite transférées dans une poche contenant 500 ou 750 ml de milieu C, puis ensemencées dans deux ou trois unités double-plateaux (double-tray de Nunc) d'une surface totale de 1200 ou 1800 cm². Les cellules sont mises à incuber. Une troisième expansion est décidée lorsque le degré de confluence des cellules atteint 60% à 70% ou lorsque les premiers myotubes apparaissent. Pour cette série d'expansions, les boîtes multi-étagées de 10 plateaux (multi-tray de Nunc) sont utilisées. Après vidange du milieu, les cellules sont lavées à l'aide de 100 ml de solution D. Le décollement de la couche cellulaire et la dissociation enzymatique des cellules sont réalisés après vidange de la solution de lavage par l'addition de 50 ml de trypsine irradiée (0.25%) dans chaque plateau. Les préparations sont mises à incuber 5 minutes à 37°C. Les cellules sont récoltées dans des poches stériles de 300 à 600 ml. L'action de la trypsine est neutralisée par l'addition de 10% en volume de sérum de veau foetal. Les cellules sont centrifugées et le surnageant est éliminé par connexion à une poche de vidange.

Le culot cellulaire est resuspendu dans 50 ml de milieu C puis transféré dans un ou deux flacons contenant 1200 ou 2400 ml de milieu C par un connecteur stérile. Une ou deux boîtes de culture de 10 plateaux multi-étagés (multi-tray de Nunc) est ensemencée avec 1200 à 2400 ml de la préparation cellulaire. Le contrôle visuel de la croissance en plateaux multi-étagés est impossible. Par conséquent, une boite de culture Nunc stérile simple plateau (single-tray) est ensemencée aussi avec 110 ml de la préparation cellulaire. Ce dernier plateau permet un contrôle visuel quotidien de la culture et de l'état de confluence des cellules. Les cellules sont ensuite mises à incuber. La culture dure de 3 à 5 jours. La veille de la récolte des cellules, le milieu est éliminé par vidange dans une poche stérile et remplacé par un volume équivalent de milieu E. La récolte finale est décidée lorsque le degré de confluence des cellules atteint 90% ou lorsque les premiers myotubes apparaissent.

### A.2.4 identification des types cellulaires présents aux différents stades de la culture par l'analyse de marqueurs cellulaires spécifiques

La caractérisation est basée sur l'analyse par cytofluorimétrie de flux (FACS). Des anticorps dirigés contre les antigènes humains de surface cellulaire suivants ont été utilisés : CD5, CD10, CD11, CD13, CD14, CD15, CD16, CD18, CD19, CD20, CD28, CD31, CD34, CD38, CD40, CD40-ligand, CD44, CD45, CD56, CD62, CD71, CD80, CD86, CD90, CD105, CD117, CD138. Des anticorps dirigés contre les structures antigéniques suivantes ont aussi été utilisés: CD138, HLA-Classe I, HLA-DR, ELAM, ICAM, LECAM, Stro-1, S-endo-1, VCAM, VLA2, VLA3, VLA4, VLA5, VLA6.

L'analyse de l'expression de la desmine, protéine intracellulaire est réalisée de la manière suivante :
Après mise en suspension dans du PBS, les cellules sont fixées et perméabilisées par l'addition de 10 volumes de méthanol à 4°C pendant 5 minutes, puis centrifugées. Après lavage du méthanol résiduel et centrifugation, les cellules sont reprises dans du PBS contenant l'anticorps dirigé contre la desmine (Dako, clone D33, 1/100) et incubées durant 15 minutes. Après lavage et centrifugation, les cellules sont incubées durant 15 minutes en présence de l'anticorps secondaire dirigé contre l'anticorps primaire, couplé à un fluorophore. Après lavage et centrifugation, les cellules sont analysées par FACS. Le tableau I ci-après présente les caractéristiques des marqueurs principaux utilisés et les types cellulaires correspondants (types cellulaires).

### CARACTERISTIQUES CLASSIQUES DES MARQUEURS UTILISES

**Tableau I : Présentation de certains marqueurs cellulaires utilisés dans le procédé et les types cellulaires les exprimant.**

| **MARQUEUR** | **TYPES CELLULAIRES** |
|---|---|
| CD10 | lymphocytes préB. neutrophiles |
| CD13 | monocytes, cellules myéloïdes |
| CD15 | monocytes, macrophages, granulocytes, éosinophiles |
| CD16 | NK, sous pop. lymphocytes T, neutrophiles |
| CD34 | progéniteurs |
| CD38 | LT activités, cellule souche, sous pop. L T,B,NK |
| CD40 | lymphocytes T CD4+ activés |
| CD44 | Anti HCAM |
| CD45 | leucocytes |
| CD56 | NK, sous pop. Lymphocytes T |
| CD71 | cellules proliférantes |
| CD117 | progéniteurs |
| HLA CL1 | antigène MHC classe 1 |
| HLA CL2 | antigène MHC classe 2 |
| VLA3 | lymphocytes B |
| VLA5 | lymphocytes T mémoire, monocytes, plaquettes |
| VLA6 | thymocytes, lymphocytes T mémoire, monocytes |
| DESMINE | cellules musculaires |

L'analyse des populations cellulaires est réalisée aux différents stades de culture :
- Au jour J0 correspondant à la mise en culture de la population fraîchement préparée.

Plusieurs séries ont été réalisées en flacons unitaires mis en culture parallèlement à l'unité simple plateau à J0, afin de pouvoir suivre quotidiennement l'évolution des cultures en prélevant un ou plusieurs flacons par jour.
- Au jour 1 (J1), la fraction non adhérente (surnageant) et la fraction adhérente (obtenue par trypsination) ont été analysées indépendamment. De manière intéressante, il a été montré que ces deux fractions renferment des populations aux caractéristiques différentes.
- De J2 à J9, les cellules adhérentes ont été analysées chaque jour afin de suivre l'évolution des types cellulaires au cours du temps.

En parallèle, les cellules obtenues par production en masse ont été analysées à chaque étape d'expansion puis au moment de la récolte finale. Les résultats ont montré qu'à date de récolte équivalente, les caractéristiques obtenues dans les plateaux et les flacons indépendants sont identiques.

Les données issues de l'identification des types cellulaires au cours de la cinétique de différenciation sont présentées dans les tableaux JA et JB suivants et les caractéristiques essentielles sont décrites ci-après.

### Cinétique de différenciation (pourcentages)

**Tableau JA : Identification des types cellulaires au cours du temps (en pourcentage dans la population cellulaire totale) des stades J0 à J3 de la culture.**

| **MARQUEURS** | **J0** | **J1** | **SN J1** | **J2** | **J3** |
|---|---|---|---|---|---|
| CD34+ | 38,5 (25-75) | 77 (69-79) | 39 (25-67) | 74 (64-75) | 74 (51-75) |
| CD34- | 61,5 (25-75) | 23 (20-30) | 61 (33-75) | 26 (25-30) | 26 (25-49) |
| CD34+CD10+ | 14 (10-18) | 27 (25-29) | 2,5 (2-3) | 24 (20-40) | 11 (7-17) |
| CD34+CD10- | 25 (23-31) | 47 (40-54) | 30,5 (24-37) | 39 (36-54) | 59 (44-63) |
| CD34+CD45+ | <5 | ND | 0,5 (0-1) | ND | ND |
| CD34+CD56+ | 0 | 0 | 0 | 0,3 (0,3-0,3) | 0,7 (0,4-2) |
| CD34+DR+ | 14 | ND | 21,5 (16-27) | ND | ND |
| CD34+DR- | 28 | ND | 12 (12-12) | ND | ND |
| CD34+CD 15+ | ND | ND | ND | ND | ND |
| CD56+ | 4,7 (0-26) | 4,4 (0,3-15) | 1 (0-5) | 11 (7,7-20) | 14 (12-35) |
| CD15+ | 1,5 (0-6) | 3 (1,5-6) | 3,6 (0,1-12) | 20,5 (20-21) | 25 (24-33) |
| CD56+CD15+ | 0,02 (0-0,3) | 0 | 0 | 4 (4-4) | 10 (6-14) |
| CD13+ | ND | ND | ND | ND | ND |
| CD44+ | 0 | ND | ND | ND | ND |
| CD117+ | 1,7 (0,16-1,7) | ND | 1,7 (0,2-2) | ND | ND |

**Tableau JB : Identification des types cellulaires au cours du temps (en pourcentage dans la population cellulaire totale) des stades J4 à J8 de la culture.**

| **MARQUEURS** | **J4** | **J5** | **J6** | **J7** | **J8** |
|---|---|---|---|---|---|
| CD34+ | 56 (36-67) | 35 (26-50) | 23 (13-47) | 2,7 (0,6-10) | 1,7 (0,4-3,4) |
| CD34- | 44 (33-63) | 65 (50-73) | 76 (53-87) | 97 (90-99) | 98 (96-100) |
| CD34+CD10+ | 9 (8-22) | 16,5 (3-34) | 16,8 (0,6-33) | 1,7 (0,2-2,7) | 0,4 (0,2-7,6) |
| CD34+CD10- | 35 (27-59) | 25 (15-35) | 14 (14-14) | 5,1 (0,4-9) | 1,3 (0,2-7,6) |
| CD34+CD45+ | ND | ND | ND | ND | ND |
| CD34+CD56+ | 3 (1,5-6) | 7,5 (1,5-12) | 3 (0,1-12) | 0,35 (0,2-0,6) | 0,4 (0,4-2,1) |
| CD34+DR+ | ND | ND | ND | ND | ND |
| CD34+DR- | ND | ND | ND | ND | ND |
| CD34+CD15+ | ND | ND | ND | ND | ND |
| CD56+ | 34 (28-55) | 61 (50-68) | 73 (57-74) | 64 (52-87) | 68,4 (50,3-91) |
| CD15+ | 48 (46-48) | 48 (47-63) | 51 (29-64) | 36 (20-55) | 29 (12-54) |
| CD56+CD15+ | 19 (17-21) | 13 (9-25) | 10(5-13) | 3,6 (2,6-6) | 1,4 (1-3) |
| CD13+ | ND | ND | 96 (76-97) | 99 (94-99) | 99 (96-99) |
| CD44+ | ND | ND | ND | ND | ND |
| CD117+ | ND | ND | ND | ND | ND |

### A.2.4. 1 Caractéristiques des préparations cellulaires à J0

Le procédé permet d'obtenir à J0 3x10⁵ à 4X10⁶ cellules par gramme de tissu. Les types cellulaires majoritaires sont CD34+. Les types cellulaires CD34+ sont CD34+/CD10+ à 14%; CD34+/CD10- à 25% ; CD34+/CD56+ à 0% ; CD34+/DR+ à 14% et CD34+/DR- à 28%. Les populations CD34+ sont CD45- en majorité. Des populations minoritaires expriment CD44, CD45, CD56, CD117, HLA-Classe Il. De manière surprenante, la caractérisation d'un nombre de cellules progénitrices important (en quantités absolue et relative dans la population) permet d'envisager, par une récolte des cellules à ce stade, leur utilisation comme produit de thérapie cellulaire pour la reconstitution de nombreux tissus, non seulement musculaires mais aussi hématopoïétiques, osseux, adipeux, cartilagineux ou vasculaires.

### A.2.4.2 Caractéristiques des préparations cellulaires adhérentes à J1

Une majorité de cellules est CD34+. La population se compose de CD34+/CD10+ (27%) et CD34+/CD10- (47%). CD13 apparaît. La préparation est négative pour CD117 et CD45. Les types cellulaires CD15+ et CD56+ sont minoritaires.

### A.2.4.3 Caractéristiques des préparations cellulaires présentes dans le surnageant à J1.

Une proportion importante de cellules est CD34+. La population se compose essentiellement de CD34+/CD10-. Une population CD117+ (<5%) est présente et exprime CD45+. Certaines populations minoritaires sont présentes: CD38+ (15%), CD45+, peu de CD15+ et de CD56+, HLA-Classe II+.

### A.2.4.4 Caractéristiques des évolutions des marqueurs au cours de la culture

L'évolution est marquée par une augmentation croissante de la proportion de cellules CD15+ et/ou CD56+ et une diminution des populations CD34+. On peut observer au cours du temps la transition progressive d'une population de CD34+ à CD15+. La proportion des populations CD13+, CD44+, CD71+ augmente avec le temps. On observe des populations minoritaires CD138+, HLA-Classe II+, CD38+.

En fin d'expansion, on observe trois populations prépondérantes : CD56+, CD15+ et CD56-/CD15-. La population CD56+ exprime CD10, CD13, CD44, la desmine et HLA-Classe I. Ces marqueurs sont spécifiques des cellules myoblastiques. La population CD15+ exprime CD13, Classe I et partiellement CD10. Dans la population CD56-/CD15-, une fraction exprime la desmine et l'autre fraction ne l'exprime pas. Certains marqueurs sont exprimés plus faiblement et de façon variable : CD71, VLA3, VLA5, VLA6, CD16+ et CD40L. Les populations CD34+, CD38+, CD45+ et HLA-Classe II+ ont disparu ou sont extrêmement minoritaires.

### A.2.4.5 Caractéristiques des cellules après déplétion de la fraction CD34+

Le tableau K ci-dessous représente les caractéristiques des cellules obtenues, à l'issue du premier passage, en comparant différentes conditions initiales. Huit expériences indépendantes sont représentées. Après déplétion de la fraction CD34+, le procédé permet d'obtenir rapidement une population cellulaire très majoritairement composée de cellules exprimant le CD56. En particulier, la proportion de cellules exprimant CD56 est supérieure à celle obtenue à partir d'une biopsie non déplétée.

**Tableau K. Déplétions ou enrichissements en cellules CD34+ présentes au sein de la biopsie musculaire.**

| | %CD56+ au premier passage | | | | | | | |
|---|---|---|---|---|---|---|---|---|
| | Expérience 1 | Expérience 2 | Expérience 3 | Expérience 4 | Expérience 5 | Expérience 6 | Expérience 7 | Expérience 8 |
| Fraction Non Séparée | ND | 72% | 87 % | 41 % | 48 % | 67 % | 70% | 38 % |
| Fraction Déplétée en CD34 | 93 % | 98 % | 97 % | 82% | 93% | 93 % | 86% | 57 % |
| Fraction Enrichie en CD34 | 61 % | 36% | 37 % | 1% | 8% | 28 % | 4 % | 12% |

### A.2.5 Récolte des cellules

Le protocole suivant décrit la récolte des cellules au stade final pour l'obtention d'une population myoblastique majoritaire. Cependant, le protocole permet à l'homme du métier de le mettre en oeuvre quelque soit le stade de différenciation choisi pour la récolte des cellules et ce, en fonction de la population cellulaire recherchée.

Après vidange du milieu, les cellules sont lavées à l'aide de 500 ml de solution D (pour les unités multi-plateaux), 50 ml pour l'unité simple et 100 ml pour les unités doubles. La solution de lavage est vidangée et 200 ml de solution de trypsine irradiée (0.25%) sont ajoutés aux unités multi-plateaux (20 ml pour l'unité simple, 40 ml pour les unités doubles). La préparation est mise à incuber 5 minutes à 37°C. Les cellules sont récoltées par vidange dans une poche de 500 ml. L'action de la trypsine est neutralisée par l'addition de 10% de sérum de veau foetal injecté par une seringue. Les cellules sont lavées de la manière suivante : les cellules sont centrifugées. Le surnageant est éliminé puis les cellules sont remises en suspension dans 300 ml de solution F puis centrifugées. Les surnageants sont éliminés. Deux autres étapes de lavages comme précédemment décrit sont réalisées. Ces lavages successifs ont pour but d'éliminer la trypsine, les protéines animales encore présentes et le bFGF recombinant. Au cours du troisième lavage, une aliquote est réservée pour le comptage cellulaire, l'estimation de la viabilité et de la qualité cellulaire, les contrôles de qualité microbiologique. Les cellules peuvent être concentrées dans la solution H de manière à obtenir une suspension adéquate pour l'usage clinique demandé.

Après centrifugation, les cellules sont reprises dans un volume de solution isotonique à une concentration de 1.5x10⁸ cellules/ml. Elles sont enfin aspirées dans une seringue de 10 ml. Les cellules sont prélevées pour l'injection dans des seringues stériles. Le type d'aiguille utilisée pour l'injection dépend du tissu ciblé. Pour l'injection intramyocardique directe, une aiguille coudée à 90° de 25 à 30 gauge est spécifiquement utilisée.

### A.2.6 Rendements de production et caractéristiques des types cellulaires

Le tableau L ci-dessous récapitule les résultats obtenus lors de la mise en oeuvre du procédé de l'invention à partir de différentes biopsies obtenues de trois patients différents.

Les cellules ont été produites initialement dans les unités de simples, doubles et multiples plateaux jusqu'à la troisième expansion incluse. Les expansions ont par la suite été réalisées par dédoublement des populations et repiquages dans des boites de culture de 25 cm². A chaque passage, la majorité des cellules était utilisée pour la caractérisation et le comptage, tandis qu'un nombre connu de cellules était utilisé pour l'ensemencement de dédoublement. Le nombre de dédoublements cumulés permet, par le calcul, d'obtenir environ 100 milliards de cellules entre la huitième et la neuvième expansion.

Le tableau L présente les rendements en terme de nombre de cellules obtenues et en terme de proportion de cellules du type CD56+ ou desmine+ dans la population aux différentes phases d'expansion pour les trois patients nommés MYO 003, MYO 004 et MYO 005.

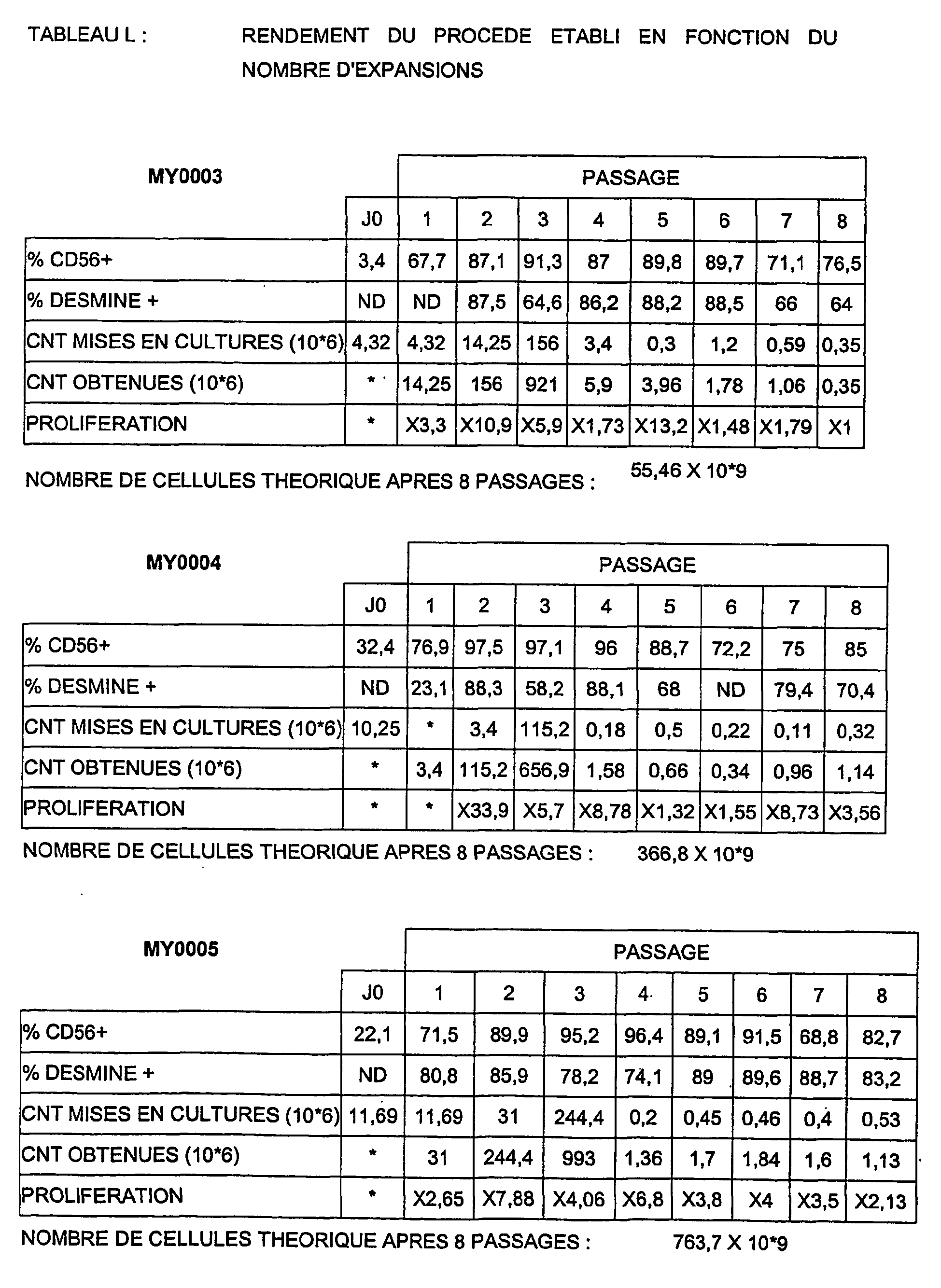

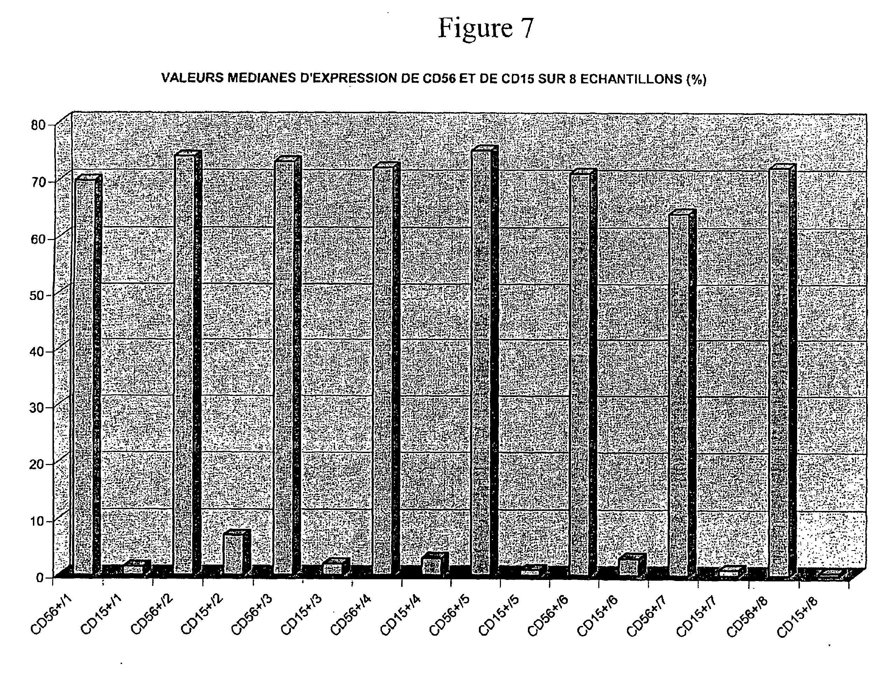

L'histogramme à la figure 7 présente les valeurs médianes d'expression de CD56 et de CD15 sur 8 échantillons au cours des différentes phases d'expansions.

Les résultats présentés à la figure 7 montrent que les proportions des types cellulaires CD56+ et CD15+ obtenues sont relativement homogènes selon les différentes biopsies, et ne sont pas modifiées au cours des différentes expansions. En particulier, on constate que les cellules de type CD56+ restent en proportion dominantes au cours des phases d'expansion.

Ces résultats montrent en outre, que après identification des stades de récoltes optimaux en fonction des types cellulaires recherchés, l'homme du métier peut reproduire le procédé de l'invention en s'affranchissant de l'étape de caractérisation cellulaire telle que définie dans le procédé de l'invention et en multipliant les phases d'expansion de manière à obtenir un grand nombre de cellules comprenant un type cellulaire spécifique en proportion dominante, et notamment le type cellulaire CD56+.

### A.2.7 Congélation du produit de thérapie cellulaire

Afin de permettre l'utilisation dans le temps des cellules ainsi préparées, il peut être avantageux de les congeler dans des conditions telles que la décongélation ultérieure permette une survie des cellules suffisante, de préférence supérieure à 90%.

A titre d'exemple, les cellules sont suspendues dans le milieu de congélation (solution G) et transférées dans deux poches de congélation stériles, à une concentration comprise entre 10⁷ et 2x10⁷ cellules/ml ou dans des tubes de cryocongélation à une concentration comprise entre 1 x 10⁶ et 5 x 10⁶/ml. La congélation est réalisée à l'aide d'un dispositif (Digicool ou Nicool) assurant une descente progressive en température contrôlée. Les cellules sont stockées dans l'azote liquide jusqu'au moment de la décongélation.

La décongélation des cellules est réalisée au bain-marie à 37°C. Les préparations cellulaires sont lavées deux fois à l'aide d'une solution saline isotonique. Les rinçages sont effectués par connexion stérile aux poches de solution isotonique et aux poches de vidange. Une aliquote est réservée pour l'estimation de la viabilité et de la qualité cellulaire.

### B. FACTEURS AFFECTANT LA FONCTIONNALITE DE LA TRANSPLANTATION DE CELLULES D'ORIGINE MUSCULAIRE AUTOLOGUES POUR LE TRAITEMENT DE MODELES D'ISCHEMIES MYOCARDIQUES

### B.1 Matériels et méthodes

### B.1.1 Modèle d'ischémie myocardique

Des rats mâles Wistar, pesant 280 g ont été anesthésiés à la kétamine (50 mg/kg) et la xylasine (10 mg/kg) et ventilés par la trachée. Une thoracotomie a été réalisée. L'infarctus du myocarde est obtenu par ligature coronaire gauche en utilisant un fil de polypropylène 7/0.

### B.1.2 Tests de fonctionnalité

Une semaine après l'infarctus du myocarde, et un ou deux mois après transplantation, la fonction ventriculaire gauche a été étudiée par échocardiographie bi-dimensionnelle (2D).

Sous anesthésie générale à la kétamine (50 mg/kg) et la xylasine (10 mg/kg), les mesures 2D (et M-mode) sont réalisées avec un appareil commercial de 15MHz (15L8) « linear-array transducer » (Sequoia, Acuson Corp., Mountain View, CA, USA) autorisant une fréquence maximale de 160 Hz. Des vues parasternales longitudinales sont obtenues, de manière à ce que les valves mitrales et aortiques et l'apex puissent être correctement visualisées et ainsi enregistrées.

Les mesures des longueurs (L) du grand axe du ventricule gauche et les tracés des zones endocardiaques (a) sont réalisées. Le volume en fin de diastole du ventricule gauche (LVEDV) et le volume en fin de systole du ventricule gauche (LVESV) ont été calculés avec la formule suivante : V = 8x A² / (3 x π x L). Les fractions d'éjection du ventricule gauche (LVEF) sont ainsi calculées : LVEF=(LVEDV-LVESV)/LVEDV. Toutes les mesures ont été faites à partir d'au moins trois battements et à l'aide de deux expérimentateurs traitant les différents groupes en aveugle.

### B.1.3 Culture des cellules

Pendant la procédure d'infarctus du myocarde, les muscles antérieurs des tibiaux droit et gauche sont disséqués de manière à séparer le tendon et le tissu aponeurotique du tissu musculaire. Ils sont ensuite émincés, pesés et dissociés à l'aide d'enzymes en utilisant la collagénase IA (2 mg/ml, Sigma Chemical Co., St. Louis, MO, USA) pendant une heure et la trypsine-EDTA (0.25%, GIBCO BRL, Gaithersbueg, MD, USA) pendant 20 minutes.

Les cellules sont récoltées par sédimentation (7 min à 1200 rpm) et la réaction enzymatique est neutralisée par l'addition de 10% de sérum de veau foetal. Après passage sur un tamis de 100 µm et centrifugation, le surnageant est éliminé et les cellules sont resuspendues dans un milieu composé de F12(HAM) avec 20% de sérum de veau foetal, 1% (vol/vol) penicilline-streptomycine (10000 Ul/ml-10000 µg/ml, GIBCO BRL) et 5 ng/ml de bFGF (Sigma).

L'ensemencement initial est réalisé en flacons de culture de 75 cm² et les cellules sont incubées en air saturé en humidité à 5% de CO₂.

Le jour de la transplantation, après 7 jours de culture et après évaluation fonctionnelle du niveau de base des fractions d'éjection ventriculaire par échocardiographie, les cellules ont été récoltées par trypsination, lavées et la viabilité a été testée. Un échantillon a été ensemencé sur des boîtes 12-puits dans 2.0 ml de milieu de culture pour comptage. Les cellules sont lavées dans le milieu d'injection (milieu de culture + BSA à 0.5%, Fraction V) et gardées dans la glace avant transplantation. Les cellules sont centrifugées, resuspendues dans 150 µl de milieu d'injection et administrées par voie sous-épicardique dans la zone infarcie.

### B.1.4 Transplantation des cellules dans la zone infarcie

Quarante-quatre rats ont fait partie de cette étude et ont été divisés en deux groupes : Un groupe contrôle et un groupe traité.

Tous les rats ont été réopérés une semaine après l'infarctus du myocarde, sous anesthésie générale et ventilation de la trachée. Tous les rats ont reçus 150 µl de milieu d'injection administré dans la zone infarcie au moyen d'une aiguille 30 Gauge. Dans le groupe contrôle (n=23), les rats ont reçu le milieu d'injection seul. Le groupe traité (n=21) a reçu la suspension de cellules myogéniques cultivées.

Dans chaque groupe, quatre catégories de risques ont été étudiées par rapport à la fraction d'éjection de base LVEF : <25% (n=15), 25-35% (n=15) et >40% (n=16). Cette stratification permet d'obtenir des nombres homogènes d'animaux dans chaque sous-groupe afin de rendre les résultats statistiques plus précis.

### B.1.5 Etudes immuno- et histochimiques

Un jour après transplantation, les cellules ensemencées en boite de 12-puits sont fixées au méthanol et refroidies à -20°C pendant 5 minutes. Le marquage non spécifique est neutralisé en utilisant un mélange de 5% de sérum de cheval (HS) et 5% de sérum de veau foetal dans du PBS pendant 20 minutes. Les cellules sont incubées avec un anticorps de souris dirigé contre la desmine (1/200 DAKO, A/S-Denmark) pendant 1 heure puis avec un anticorps anti-souris conjugué au marqueur Cy3 (1/200, Jackson Immuno Research Laboratories, Inc.) pendant une heure dans l'obscurité.

Les cellules sont observées à l'aide d'un microscope inversé à contraste de phase et à illumination fluorescente. Plusieurs clichés ont été pris de manière aléatoire. La proportion de myoblastes est calculée en divisant le nombre de cellules positives à la desmine par le nombre total de cellules observées.

Dans les trois mois qui suivent la dernière échocardiographie (soit 2 mois après transplantation), les rats sont sacrifiés par une surdose de ketamine et de xylasine. Les ventricules sont isolés et sont sectionnés en deux parties le long de leur axe longitudinal. Les deux parties sont mises dans l'isopentane et congelées à l'azote. De fines sections de 8 µm sont préparées en utilisant un cryostat et des études classiques histologiques sont réalisées par coloration à l'hématoxyline et à l'éosine.

### B.1.6 Analyse statistique

Toutes les données sont en moyenne à ± 1 SEM. Toutes les analyses ont été réalisées à l'aide du logiciel approprié (Statview 5.0, SAS Institute Inc., Cary, NC, USA). Le seuil critique α pour les analyses a été choisi à p<0.05.

Les comparaisons des variables continues entre les groupes contrôle et traité et chaque catégorie de risque ont été réalisées à l'aide de l'analyse de variance (méthode ANOVA) suivi du test post hoc (Scheffé). Les études longitudinales comparant les données échocardiographiques pour chaque groupe, avant et un et deux mois après l'injection intramyoacardique ont été réalisées en utilisant le test d'appariement.

Pour tester les relations entre le nombre de cellules injectées et la fonction cardiaque après transplantation, deux variables ont été créées : (LVEF à 1 mois/ LVEF) et (LVEF à 2 mois/LVEF). Le lien a été étudié par le calcul du F-ratio pour la régression ANOVA et le coefficient R² ajusté pour les analyses de régression linéaire.

De plus, la variabilité à l'intérieur de chaque groupe des tests échocardiographiques a été observée à partir de deux lots de mesures effectuées sur 10 rats choisis de manière aléatoire en utilisant une analyse de Bland et Altman.

### B.2 Résultats

### B.2.1 Caractérisation de la suspension injectée

Sur 10000 cellules comptées le jour de la transplantation, 50% sont positives pour l'expression de la desmine. Le nombre de cellules injectées est de 3.500 000 ± 500000 s'étalant de 700000 à 6.5x10⁶.

### B.2.2 Test de fonctionnalité après transplantation des cellules d'origine musculaire

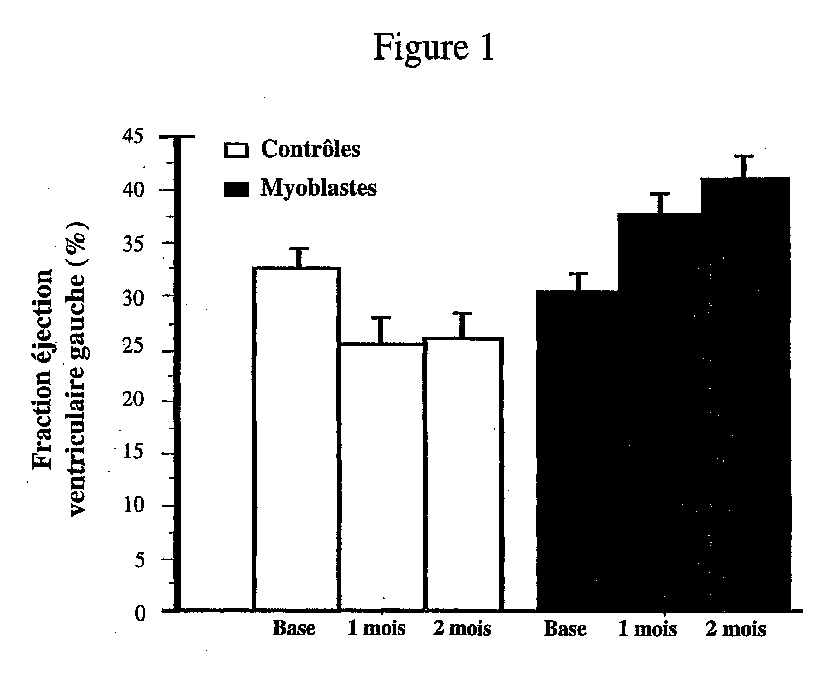

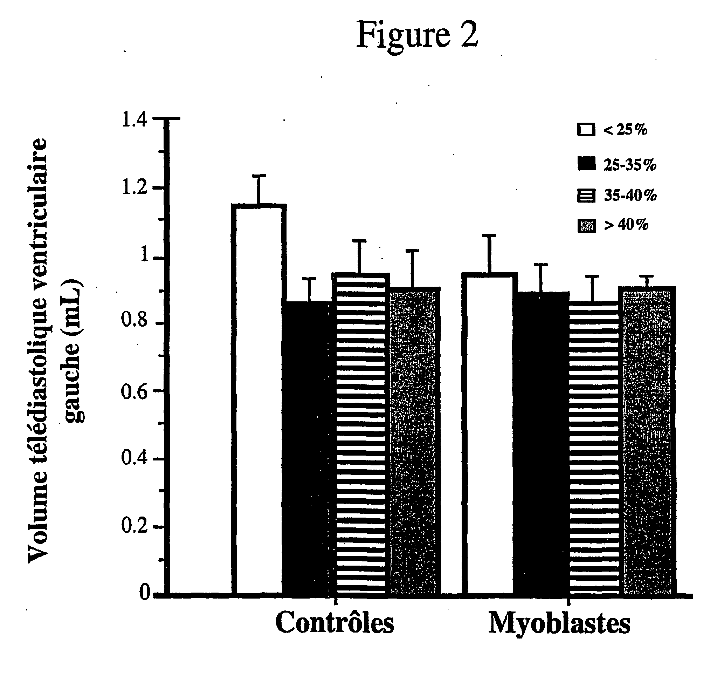

Les paramètres échocardiographiques du niveau de base ne sont pas significativement différents entre les groupes. Au contraire, des différences majeures entre les groupes sont observées après la transplantation. Ainsi, dans le groupe traité, la fonction cardiaque est améliorée, comme cela est montré par comparaison de LVEF avec les groupes contrôles (Figure 1). A un mois après l'injection myocardique, des différences significatives sont observées entre les deux groupes (37.52 ± 1.92% contre 25.49 ± 2.47%, p=0.0005). Ce résultat est confirmé après 2 mois après l'injection (40.92 ± 2.17% contre 25.83 ± 2.39%, p<0.0001). Cette amélioration de LVEF dans le groupe traité est essentiellement reliée à une plus petite augmentation de LVESV par rapport à la dilatation ventriculaire, représentée par la variable LVEDV, qui augmente de manière similaire dans les deux groupes (Figure 2).

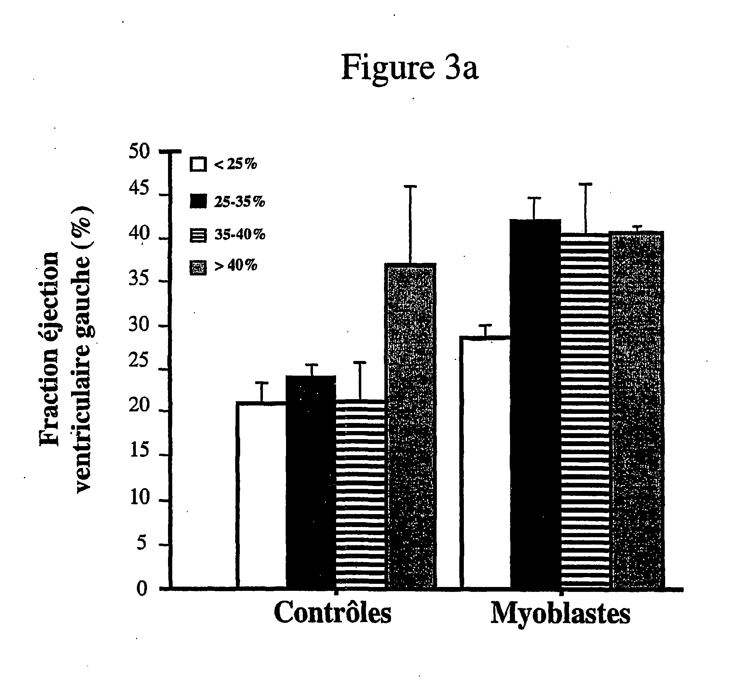

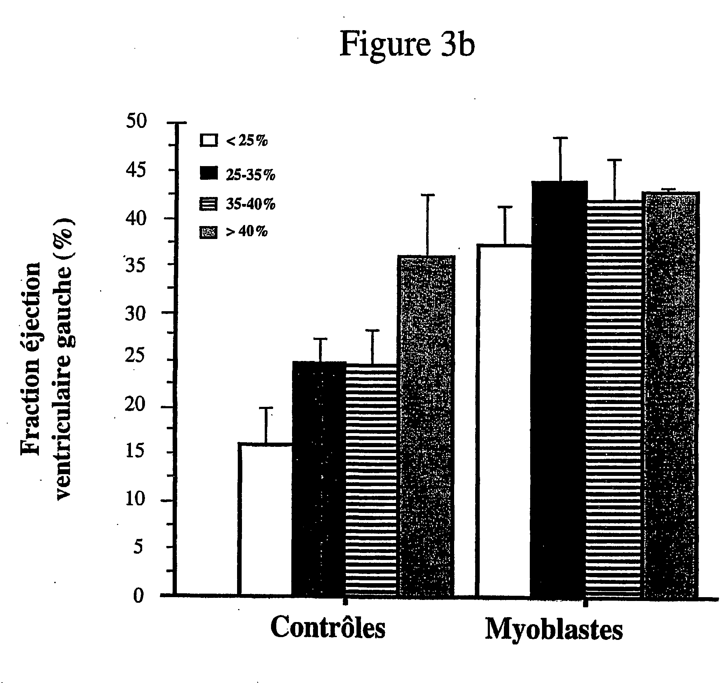

Les analyses longitudinales dans chaque groupe ont montré une amélioration substantielle de la fonction du ventricule gauche dans le groupe traité (Figure 3). Des différences significatives sont observées en comparant LVEF à un mois et LVEF à 2 mois à la variable LVEF du niveau de base. Les différences sont aussi significatives en comparant LVEF à un mois à LVEF à 2 mois. Tandis que LVEDV et LVESV sont augmentés à un mois en comparant aux valeurs de bases (p<0.0001 et p=0.0003, respectivement) et à 2 mois (p<0.0001 et p=0.029, respectivement), une stabilisation et une diminution sont montrées quand les valeurs à 2 mois sont comparées aux valeurs à 1 mois (p=0.78 et p=0.12, respectivement). Dans le groupe contrôle, une importante diminution de LVEF avec une augmentation significative de LVEDV est déjà visible à un mois (p=0.0066 et p<0.0001 contre valeurs de base respectivement). Les deux paramètres indiquent des valeurs similaires à 2 mois.

Quand la fonction cardiaque (LVEF) est analysée par catégories de risques selon les valeurs de bases LVEF, des différences sont observées entre les groupes contrôle et traité à un mois dans les deux sous-groupes intermédiaires 25-35% et 35-40%. Cette amélioration de LVEF est confirmée à 2 mois après injection dans les deux sous-groupes, mais de manière intéressante, un effet bénéfique est aussi observé, en comparant le groupe traité au groupe contrôle dans le groupe de risque <25%.

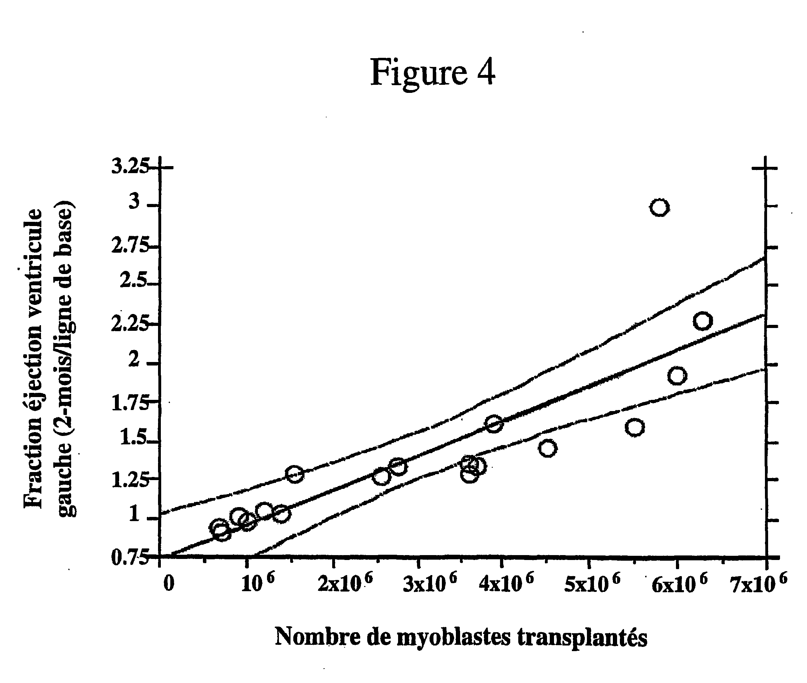

Enfin, l'étude de régression montre un lien significatif entre le nombre de myoblastes greffés et les ratios LVEF à un mois (R²=0.675, p<0.0001) et à 2 mois (R²=0.714, p<0.0001) (Figure 4). Quand les données sont analysées par groupe de risque, l'impact du nombre de cellules injectées à 2 mois est aussi significatif dans les sous-groupes <25%, 25-35% et 35-40% (R²=0.836, p=0.0106 ; R²=0.928, p=0.0083 et R²=0.985, p=0.0076, respectivement).

### B.2.3 Effets cumulés de la transplantation des cellules d'origine musculaire et d'un traitement par un inhibiteur de l'enzyme de conversion de l'angiotensine (ACEI)

La prise en charge de l'insuffisance cardiaque consiste à l'heure actuelle en l'administration d'inhibiteurs d'ACE. Il était par conséquent intéressant d'étudier s'il pouvait exister une synergie entre la transplantation de cellules d'origine musculaire obtenues selon le procédé de l'invention et l'effet protecteur procuré par les inhibiteurs d'ACE.

Un infarctus du myocarde a été réalisé chez 39 rats par ligature des artères coronaires. Un traitement au perindoprilat à 1 mg/kg par jour, un inhibiteur de l'ACE, a été initié immédiatement après l'infarctus et continué sans interruption jusqu'au sacrifice de l'animal. Une semaine après l'infarctus, les animaux ont été opérés à nouveau et sélectionnés de manière aléatoire pour recevoir une injection sous-épicardique de 150 µl de milieu de culture seul (groupe contrôle, n=21) ou un volume équivalent contenant les cellules d'origine musculaire, soit environ 3x10⁶ cellules cultivées à partir d'une biopsie de muscle de tibia antérieur et récoltées au moment de l'infarctus (groupe traité, n=18). La fonction ventriculaire gauche a été testée par échocardiographie un mois après la transplantation. Le niveau de base de la valeur de la fraction d'éjection est similaire entre les animaux contrôles (24 ± 2%) et les animaux traités (28 ± 1%) (p = 0.11). Un mois après la transplantation, les valeurs des fractions d'éjection ont augmenté dans les deux groupes et sont de 32% ± 2% pour le contrôle et de 38% ± 2% pour le groupe traité. Cependant, on constate une plus forte augmentation chez le groupe traité (p< 0.0001 contre niveau de base) par rapport au groupe contrôle (p=0.004 contre niveau de base), la valeur de fraction d'éjection étant significativement plus élevée chez les rats traités (p=0.04 contre le groupe contrôle). L'analyse des données de volume a montré que l'amélioration fonctionnelle apportée par la transplantation de cellules d'origine musculaire est essentiellement reliée à une augmentation de la contractilité plutôt qu'à une modification du ventricule gauche.

Ces données montrent qu'il existe un effet additif de la transplantation de cellules d'origine musculaire et du traitement aux inhibiteurs d'ACE.

### C. ESSAIS CLINIQUES CHEZ L'HOMME DE TRANSPLANTATION DE CELLULES D'ORIGINE MUSCULAIRE POUR LA RECONSTITUTION DES TISSUS MYOCARDIQUES

Des essais cliniques de transplantations des cellules d'origine musculaire préparées selon le procédé de l'invention ont été réalisés chez l'homme en vue de traiter l'insuffisance cardiaque.

### C.1 Méthodes

Les essais ont été réalisés chez 6 patients, dont, au moment de leur inclusion, l'âge était compris entre 18 et 75 ans. Ces patients ont tous fait l'objet d'indications cliniques de pontage aorto-coronaire avec possibilité technique de revascularisation chirurgicale. Leur fraction d'éjection globale ventriculaire gauche (mesurée par échocardiographie et/ou angiographie et/ou isotopes) était inférieure ou égale à 35%. Ils avaient tous des antécédents d'infarctus myocardique transmural. Enfin, les patients avait une hypo ou akinésie segmentaire touchant deux segments contigus ou plus (en dehors d'un anévrisme), en rapport avec l'infarctus. L'activité métabolique résiduelle dans cette zone était inférieure à 50%, détectée à la tomographie par émission de positons (TEP) et il n'y avait pas d'augmentation de cinétique à l'échographie Dobutamine à faible dose (10 gamma/Kg/mn).

Dix à dix huit grammes de tissus autologues sont prélevés au niveau du *vastus lateralis* chez le patient. L'incision est réalisée à l'aplomb du vaste externe. Le protocole de mise en culture et d'expansion des cellules est celui décrit dans la partie A du présent texte.

La culture cellulaire à injecter est ensuite déposée dans une cupule en inox stérile pour être aspirée dans une seringue de 1 ml. Un pontage coronaire est d'abord réalisé sous circulation extra-corporelle selon la technique habituelle. Après analyse de l'extension de l'infarctus et repérage des bords de la zone nécrosée, la suspension cellulaire d'environ 650 à 1200 millions de cellules (1,5x10⁸ cellules/ml) est injectée, dans et aux confins de la zone infarcie, à l'aide de la seringue de 1 ml. Plusieurs injections sont nécessaires pour déposer la totalité des cellules. Ce temps opératoire est réalisé sous circulation extra-corporelle et clampage.

### C.2 Méthodes d'évaluations et résultats fonctionnels

Ces essais ont permis de montrer la faisabilité et la sécurité de la technique chez l'homme. Par ailleurs, des tests de fonctionnalité sont réalisés par la mesure de la fonction ventriculaire gauche (segmentaire et globale) et du remodelage ventriculaire, couplée à la mesure de l'activité cellulaire métabolique. Ces mesures sont obtenues par des méthodes ultrasoniques telles que l'échocardiographie Doppler usuelle (mesure des diamètres, volumes et fractions d'éjection ventriculaire gauche), le Doppler tissulaire dans la zone infarcie et l'échocardiographie Dobutamine pour la recherche d'ischémie et de viabilité. Elles sont aussi obtenues par des méthodes isotopiques telles que la Tomographie à Emission de Positons (captation du désoxyfluoroglucose dans la zone infarcie).

Ces mesures sont réalisées en pré-opératoire et en post-opératoire au 1^{er} mois ± 8 jours et au 3^{ème} mois ± 8 jours.

Les résultats obtenus après transplantation de myoblastes sur un patient sont présentés ci-après.

Le patient est un homme de 72 ans, hospitalisé pour insuffisance cardiaque (classe III de la NYHA), consécutive à un infarctus du myocarde inférieur étendu et réfractaire au traitement médical, ce d'autant que bêtabloquants et inhibiteurs de l'enzyme de conversion ont dû être interrompus, car non tolérés. Le bilan extracardiaque note par ailleurs une insuffisance rénale modérée (créatinine : 200 mmol/L) et une occlusion carotidienne bilatérale sans retentissement fonctionnel au doppler intracrânien et ne relevant par conséquent pas d'un geste vasculaire autonome.

A l'échocardiographie, la fraction d'éjection ventriculaire gauche est à 20% avec une akinésie étendue de la-paroi inférieure et une hypokinésie antéro-latérale sévère. L'absence de viabilité dans cette paroi inférieure est démontrée par la persistance de l'akinésie sous dobutamine à faible dose. La paroi antéro-latérale, en revanche, a une réponse biphasique à la dobutamine (faible dose, puis forte dose) témoignant d'une viabilité et d'une ischémie. Ces données sont confirmées par la tomographie d'émission de positrons (TEP) au ₂fluoro¹⁸désoxyglucose (FDG) qui montre que la viabilité fait totalement défaut dans la paroi inférieure, mais qu'elle est présente dans les portions antérieure et latérale du ventricule gauche.

Sur la coronarographie, il existe une occlusion complète, proximale, de l'artère interventriculaire antérieure, avec une opacification tardive du lit d'aval par des collatérales homolatérales, une sténose serrée d'une branche diagonale haute et une occlusion proximale de l'artère coronaire droite. L'artère coronaire droite n'a que des irrégularités non significatives. Le présent patient présente donc en résumé :
(1) une altération importante de la fonction ventriculaire gauche,
(2) une cicatrice d'infarctus akinétique et métaboliquement non viable,
(3) une indication élective de pontage sur les artères autres que celles de l'infarctus.

Sous anesthésie locale, un fragment du vaste externe est prélevé du patient au travers d'une courte incision (5 cm). Les myoblastes sont produits selon le procédé de l'invention décrit en partie A. Le nombre de cellules est multiplié à l'aide de plusieurs expansions en plateaux multi-étagés permettant d'obtenir en deux semaines 800x10⁶ cellules, dont 65% de cellules CD56+. La proportion de cellules viables est supérieure à 96%.

Deux semaines après la biopsie, le patient est réhospitalisé dans le service de chirurgie cardiaque, en vue de son pontage. Compte tenu de la précarité de l'état hémodynamique après l'induction anesthésique (débit cardiaque à 1,5 Umin avec une saturation veineuse en oxygène à 55%) une contrepulsion par ballon intraaortique est instaurée de façon prophylactique. Les artères diagonale et interventriculaire antérieures sont pontées à l'aide d'un greffon veineux saphène et de l'artère mammaire interne gauche, respectivement, sous circulation extracorporelle et cardioplégie sanguine chaude, rétrograde, continue. Après réalisation des anastosomes coronaires, la zone infarcie de la paroi inférieure est aisément identifiée. Trente-trois injections de cellules, suspendues dans 5 ml d'albumine, sont faites dans et autour des foyers blanchâtres de nécrose à l'aide d'une aiguille de 27 G à angle droit spécialement conçue pour permettre la création de chenaux sous-épicardiques d'aspect pratiquement phlycténulaire. La durée du clampage aortique est de 56 min, dont 16 consacrées aux injections des cellules. Il n'y a aucun saignement à partir des sites d'injection, et la circulation extracorporelle peut être arrêtée sans difficultés. Le patient est sevré de la contrepulsion et d'un support pharmacologique par dobutamine au cours des 3 premiers jours postopératoires et sort au 8^{e} jour après des suites simples.

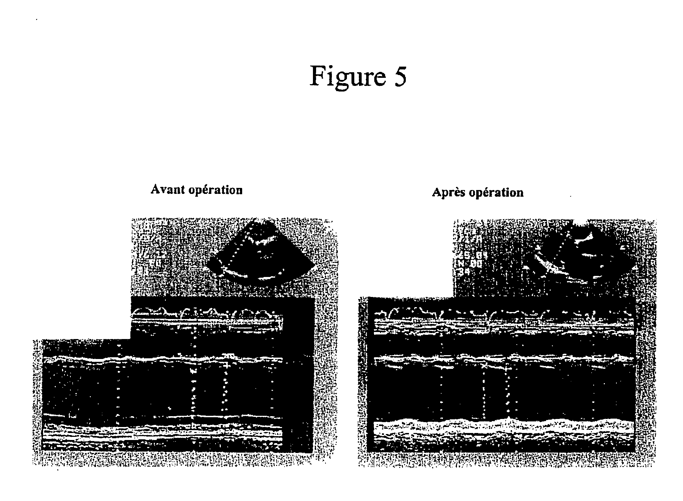

Huit mois plus tard, son état clinique s'est amélioré, et il est actuellement en classe Il de NYHA, alors que le traitement médical est resté inchangé. Un Holter des 24 heures ne montre aucun trouble du rythme. Les études de 4 échocardiographies effectuées chaque mois depuis l'opération chirurgicale montrent que la fraction d'éjection du ventricule gauche a augmenté à 30%. Comme le montre la figure 5, la contractilité segmentaire est démontrée non seulement dans la paroi antérieure, mais aussi dans la zone postérieure infarcie et greffée qui se contracte (le pourcentage d'épaississement systolique est passé de valeurs préopératoires quasiment indétectable à 40%.

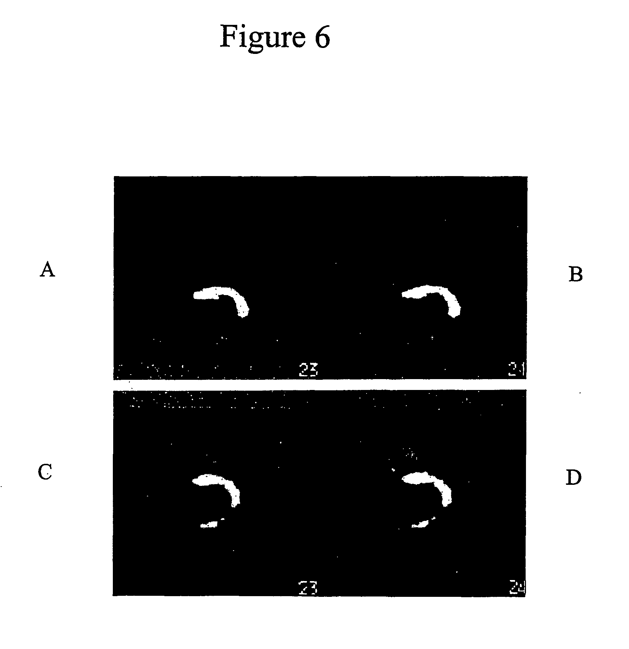

Fait nouveau, cette contractilité s'améliore encore sous dobutamine. Par ailleurs, une imagerie doppler tissulaire montre l'apparition d'un gradient de vélocité transmyocardique en systole. Une nouvelle tomographie à émission de positrons au FDG montre clairement la captation du traceur dans la paroi inférieure, avec un ratio d'activité entre la paroi et le septum (pris comme contrôle), qui augmente de 0,5 avant l'opération à 0,7, ce qui reflète une nouvelle activité métabolique dans la zone infarcie dépourvue de viabilité en préopératoire (Figure 6). Cette dernière observation ne peut avoir été influencée par la revascularisation myocardique associée et, confrontée aux données échocardiographiques, suggère que l'amélioration fonctionnelle dans la zone infarcie est réellement en rapport avec la présence des myoblastes greffés.

Pris dans leur ensemble, ces résultats montrent que la fonction de la zone infarcie a été améliorée par la transplantation de myoblastes obtenus selon le procédé de l'invention.

L'injection de cellules musculaires autologues, préparées selon le procédé, a encore été réalisée sur 5 autres patients. Les tableaux suivants résument les données du suivi clinique sur 4 des 5 patients (nommés respectivement MYO3, MYO4, MYO5 et MYO6). Le suivi clinique du sixième patient est en cours.

| Nom du patient | **D. MYO 3** |
|---|---|
| Age | 62 |
| Sexe | M |
| Type de Pathologie | Insuffisance cardiaque post-ischémique, angor instable |
| Fraction d'éjection | 25 % |
| Stade classification NYHA | III |
| PET-Scan | Présence d'une zone non viable |
| Échographie initiale | Akinésie antérieure, dyskinésie apicale, hypokinésie latérale |
| Bilan virologique | Négatif |
| Durée de culture | 18 J |
| Cellules : nombre, caractéristiques | 922 x 10⁶. CD56+ : 91 % ; CD15+ : 6 % ; Desmine+ : 65 % ; Viabilité : 98 %; Formation de myotubes (test fonctionnel) : +. |
| Siège d'injection | Antérieure ; 900 x 10⁶ cellules injectées |
| Complications infectieuses | Absence complications infectieuses post-opératoires |
| Évaluation fonctionnelle : | |
| - Stade NYHA - Fraction d'éjection - Contractilité segmentaire | - III -> II - 25 % -> 35% - Augmentation (+/-) |
| Évolution viabilité segmentaire | Amélioration (+) |

| Nom du patient | **E. MYO 4** |
|---|---|
| Age | 67 |
| Sexe | M |
| Type de Pathologie | Insuffisance cardiaque post-ischémique |
| Fraction d'éjection | 31 % |
| Stade classification NYHA | III |
| PET-Scan | Présence d'une zone non viable |
| Échographie initiale | Akinésie apicale, akinésie postérieure, hypokinésie latérale |
| Bilan virologique | Négatif |
| Durée de culture | 20 J |
| Cellules : nombre, caractéristiques | 657 x 10⁶. CD56+ : 97 % ; CD15+ : 15 % ; Desmine+ : 58 % ; HLA Classel : 94 % ; Viabilité : 98 %; Formation de myotubes (test fonctionnel) : +. |
| Siège d'injection | Postérieure ; 620 x 10⁶ cellules injectées |
| Complications infectieuses | Absence complications infectieuses post-opératoires |
| Évaluation fonctionnelle : | |
| - Stade NYHA - Fraction d'éjection - Contractilité segmentaire | - III -> II - 31 % -> 50% - Augmentation (+/-) |
| Évolution viabilité segmentaire | Amélioration (+) |

| Nom du patient | **F. MYO 5** |
|---|---|
| Age | 39 |
| Sexe | M |
| Type de Pathologie | Insuffisance cardiaque post-ischémique |
| Fraction d'éjection | 22 % |
| Stade classification NYHA | III |
| PET-Scan | Présence d'une zone non viable |
| Échographie initiale | Akinésie apicale, akinésie postérieure, hypokinésie antérieure, hypokinésie latérale |
| Bilan virologique | Négatif |
| Durée de culture | 14 J |
| Cellules : nombre, caractéristiques | 993 x 10⁶. CD56+ : 95 % ; CD15+ : 4 % ; Desmine+ : 78 % ; HLA Classel : 96 % ; Viabilité : 98 %; Formation de myotubes (test fonctionnel) : +. |
| Siège d'injection | Postéro-latérale ; 950 x 10⁶ cellules injectées |
| Complications infectieuses | Absence complications infectieuses post-opératoires |
| Évaluation fonctionnelle : | |
| - Stade NYHA - Fraction d'éjection - Contractilité segmentaire | - III -> II - 22 % -> 36% - Augmentation (+) |
| Évolution viabilité segmentaire | Amélioration (+) |

| Nom du patient | **G. MYO 6** |
|---|---|
| Age | 55 |
| Sexe | M |
| Type de Pathologie | Insuffisance cardiaque post-ischémique |
| Fraction d'éjection | 34 % |
| Stade classification NYHA | IV |
| PET-Scan | Présence d'une zone non viable |
| Échographie initiale | Akinésie antérieure, akinésie latérale, dyskinésie apicale, hypokinésie septale |
| Bilan virologique | Négatif |
| Durée de culture | 16 J |
| Cellules : nombre, caractéristiques | 1210 x 10⁶. CD56+ : 85 % ; CD15+ : 10 % ; Desmine+ : 85 % ; HLA Classel : 96 % ; Viabilité : 97 %; Formation de myotubes (test fonctionnel) : +. |
| Siège d'injection | Antérieure ; 1150 x 10⁶ cellules injectées |
| Complications infectieuses | Avant injection, patient en état de choc cardiovasculaire, sous adrénaline et noradrénaline. Absence complications infectieuses post-opératoires (24 h) après injection. |
| Évaluation fonctionnelle : | Patient décédé le 28/04/01. Cause du décès non imputable à l'injection cellulaire (choc cardio-vasculaire suivi d'ischémie mésentérique probables) |
| - Stade NYHA - Fraction d'éjection - Contractilité segmentaire | |
| Évolution viabilité segmentaire | Sans objet |

En résumé, ces essais ont montré la possibilité d'obtenir le nombre de cellules souhaité dans un délai limité de 2 à 4 semaines. Ils ont par ailleurs montré que le prélèvement, la préparation et la transplantation des cellules musculaires autologues humaines pouvaient être réalisés selon l'invention sans difficultés et complications pré, per ou post-chirurgicales.

Enfin, ces essais ont de plus permis d'observer une amélioration clinique, une augmentation de la contractilité régionale (échographie) associée à une augmentation de la surface de zone viable (PET-Scan) chez ces patients.

En conclusion, les études présentées dans les parties B et C permettent d'apporter les différents résultats suivants :
a) elles montrent que la transplantation des cellules d'origine musculaire améliore de manière significative la fonction ventriculaire gauche suite à un infarctus du myocarde,
b) que l'amélioration est clairement dépendante du nombre de cellules injectées,
c) que cette transplantation potentialise un traitement pharmacologique, notamment par une inhibition de l'ACE.
d) Et enfin que le procédé de l'invention est adapté pour le traitement de l'insuffisance cardiaque chez l'homme.

1. Emerich, D.F. (1993). Cell transplantations of Huntington's disease. Cell transplantation 4: 348.
2. Borlongan, C.V. and Sandberg, P. (1993). Microtransplantation of nigral dopamine neurons in a rat model of Parkinson's disease. Cell Transplantation 4: 347.
3. Hering, B.J., Browatzki, C.C., Schultz, A., Bretzel, R.G. and Federlin, K.F. (1993). Clinical islet transplantation - registry report, accomplishments in the past and future research needs. Cell Transplantation 4: 269.
4. Tremblay JP, Malouin F, Roy R, Huard J, Bouchard JP, Satoh A, Richards CL. (1993). Results of a triple blind clinical study of myoblast transplantations without immunosuppressive treatment in young boys with Duchenne muscular dystrophy. Cell Transplant 2: 99-112.
5. Skuk D, Roy B, Goulet M, Tremblay JP. Successful myoblast transplantation in primates depends on appropriate cell delivery and induction of régénération in the host muscle. (1999) Exp. Neuro/ 155: 22-30
6. Law PK, Bertorini TE, Goodwin TG, Chen M, Fang Q, Li H-J, Kirby DS, Florendo JA, Herrod HG, Golden GS. (1990) Dystrophin production induced by myoblast transfer therapy in Duchenne muscular dystrophy. Lancet 336: 114-115.
7. Huard J, Bouchard JP, Roy R, Malouin F, Dansereau G, Labrecque C, Albert N, Richards CL, Lemieux B, Tremblay JP. (1992). Human myoblast transplantation : preliminary results of 4 cases. Muscle Nerve 15: 550-560.
8. Dhawan, J., Pan, L.C., Pavlath, O.K., Travis, M.A., Lanctot, A.M. and Blau, H.M. (1991). Systemic delivery of human growth hormone by injection of genetically engineered myoblasts. Science 254: 1509-1512.
9. Dai, Y., Roman, M., Navlaux, R.K. and Verma, I.M. (1992). Gene therapy via primary myoblasts: long-term expression of factor IX protein following transplantation in vivo. Proc. Natl. Acad. Sci. USA 89: 10892-10895.
10. Jiao, S, Gurevich, V. Wolff, J.A. (1993). Long-term correction of rat model of Parkinson's disease by gene therapy. Nature 362: 450-453
11. Gussoni, E, Blau, HM, Kunkel, LM. (1997). The fate of individual myoblasts after transplantation into muscles of DMD patients. Nature Medicine 3:970-977.
12. Murry CE, Wiseman RW, Schwartz SM and Hauschka SD. (1996) Skeletal myoblast transplantation for repair of myocardial necrosis. J. Clin Invest 98: 2512-2523.
13. Taylor, D.A., Atkins, B.Z., Hungspreugs, P., Jones, T.R., Reedy, M.C. et al. (1998). Regenerating functional myocardium: improved performance after skeletal myoblast transplantation. Nat. Med. 4: 929-33.
14. Chiu, R.C.-J., Zibaitis, A. and Kao R.L. (1995). Cellular cardiomyoplasty: myocardial régénération with satellite cell implantation. Ann. Thorac. Surg. 60: 12-18.
15. Gussoni E, Soneoka Y, Strickland CD, Buzney EA, Khan MK, Flint AF, Kunkel LM, Mulligan RC. (1999). Dystrophin expression in the mdx mouse restored by stem cell transplantation. Nature 401:390-394.
16. Jackson KA, Mi T, Goodell MA. Hematopoietic potential of stem cells isolated from murine skeletal muscle. (1999) Proc Natl Acad Sci USA 96:14482-14486.
17. Young, H.E., Steele, T.A., Bray, R.A., Detmer, K., Blake, L.W., Lucas, P.W., Black, A.S. (1999). Human pluripotent and progenitor cells display cell surface cluster differenciation markers CD10, CD13, CD56, and MHC Class-1. Proc Soc Exp Biol Med 221: 63-71
18. PCT WO 98/54301 (Mickle, D.A., Weisel, R.). Transplants for myocardial scars and method and cellular preparations therefor.
19. PCT WO 96/18303 (Law, P.K.). Myoblast therapy for mammalian disease.
20. EP 0 898 967 A1 (Law, P.K.). Myoblast transfer therapy for relieving pain and for treating behavioural and perceptive abnormalities.
21. Webster, C., Pavlath, G.K., Parks, D.R., Walsh, F.S. and Blau, H.M. (1988). Isolation of human myoblasts with the fluorescence-activated cell sorter. Exp Cell Res 174: 252-265.
22. Pittenger, M.F., Mackay, A.M., Beck, S.C., Jaiswal, R.K., Douglas, R., Mosca, J.D., Moorman, R.K., Simonetti, D.W., Craig, S., and Marshak, D.R. (1999). Multilineage potential of adult human mesenchymal stem cells. Science 284: 143-
23. Ham RG, StClair JA, Webster C, Blau HM. (1988) Improved media for normal human muscle satellite cells: serum-free clonal growth and enhanced growth with low serum. In Vitro Cell Dev Biol 24: 833-844.
24. Robinson S.W., Cho, P.C., Levitsky, H.I., Olson, J.L., Hruban, R.H., et al. (1996). Arterial delivery of genetically labelled skeletal myoblasts to the murine heart: long-term survival and phenotypic modification of implanted myoblasts. Cell Transplant 5: 77-91.
25. Zhuquing Qu et al. Development of approaches to improve cell survival in myoblast transfer therapy. J. Cell Biol. 142: 1257-1267.
26. Lequerica et al. In vitro proliferation, differentiation and immunomagnetic bead purification of human myoblast. Annals of transplantation (1999) 4: 103-108
27. WO 99/56785 (Université Pittsburg, 1999-11-11).

## Revendications

1. Procédé de préparation d'une composition destinée à la thérapie cellulaire chez l'homme, ledit procédé comprenant les étapes suivantes :
a) éminçage d'une biopsie de tissu musculaire squelettique,
b) dissociation enzymatique des fibres et cellules musculaires et séparation des cellules individualisées par filtration,
c) culture des cellules obtenues à l'étape b) dans un réacteur de culture de cellules adhérentes en présence d'un milieu comprenant MCDB 120 et la D-valine substituée à la L-valine, jusqu'à obtention d'une population de cellules comprenant des cellules CD56+ comme type cellulaire dominant, ledit type cellulaire étant dominant lorsque la proportion de ce type cellulaire dans ladite population de cellules dépasse 50%, ladite culture comprenant le cas échéant une ou plusieurs phases d'expansion,
d) récolte de la population de cellules obtenue à l'étape c),
e) le cas échéant, congélation de la population de cellules récoltées à l'étape d).

2. Procédé selon la revendication 1, **caractérisé en ce que** la population de cellules récoltées comprend de 50 x 10⁶ cellules à 800 x 10⁹ cellules, de préférence au moins 500 x 10⁶ cellules, parmi lesquelles les cellules myoblastiques exprimant le marqueur CD56 constituent ledit type cellulaire dominant.

3. Procédé selon la revendication 2, **caractérisé en ce que** la population de cellules récoltées comprend au moins 50%, de préférence 60%, et mieux 70% de cellules myoblastiques exprimant le marqueur CD56.

4. Procédé selon l'une quelconque des revendications 2 à 3, **caractérisé en ce que** ledit milieu contient un glucocorticoïde et du bFGF.

5. Procédé selon l'une quelconque des revendications 2 à 4, **caractérisé en ce que** la composition est destinée à la reconstitution chez l'homme d'un tissu musculaire squelettique, cardiaque ou viscéral.

6. Procédé selon l'une quelconque des revendications 2 à 5, **caractérisé en ce que** la composition est destinée au traitement, chez l'homme de l'insuffisance cardiaque post-ischémique.

7. Procédé selon l'une quelconque des revendications 2 à 6, **caractérisé en ce que** la composition est destinée au traitement d'une dystrophie musculaire innée ou acquise.

8. Procédé selon l'une quelconque des revendications 2 à 7, **caractérisé en ce que** ladite étape d'éminçage est assistée à l'aide de broyeurs à couteaux, mécaniques ou électriques.

9. Procédé selon l'une quelconque des revendications 2 à 8, **caractérisé en ce qu'**il comprend en plus une étape de déplétion des cellules CD34+.

10. Produit de thérapie cellulaire pour son utilisation dans le traitement d'une pathologie cardiaque chez l'homme, comprenant une population de 500.10⁶ à 800.10⁹ cellules, ladite population comprenant au moins 50% de cellules CD56+.

11. Produit de thérapie cellulaire selon la revendication 10, **caractérisé en ce que** ladite population comprend au moins 60% de cellules CD56+.

12. Produit de thérapie cellulaire selon la revendication 10, **caractérisé en ce que** ladite population comprend au moins 70% de cellules CD56+.

13. Produit de thérapie cellulaire selon l'une quelconque des revendications 10 à 12, **caractérisé en ce que** ledit traitement d'une pathologie cardiaque est un traitement par réparation d'un tissu cardiaque chez l'homme.

14. Produit de thérapie cellulaire selon l'une quelconque des revendications 10 à 13, **caractérisé en ce que** ladite pathologie cardiaque est une insuffisance cardiaque post-ischémique.

15. Produit de thérapie cellulaire selon la revendication 14, **caractérisé en ce que** ledit traitement d'une insuffisance cardiaque post-ischémique est réalisé concomitamment à un traitement par un inhibiteur de l'enzyme de conversion de l'angiotensine (ACEI).

16. Produit de thérapie cellulaire selon l'une quelconque des revendications 10 à 15, **caractérisé en ce que** ledit traitement d'une pathologie cardiaque est un traitement par transplantation autologue.

17. Utilisation d'une population cellulaire telle que définie à l'une quelconque des revendications 10 à 16, pour la production d'un produit de thérapie cellulaire destiné au traitement d'une pathologie cardiaque.

18. Milieu de culture comprenant MCDB 120 et la D-valine substituée à la L-valine.

19. Milieu de culture selon la revendication 18, qui ne comprend pas de rouge de phénol et ne comprend pas de thymidine.

20. Utilisation in vitro du milieu de culture selon la revendication 18 ou 19, pour la différenciation de cellules de tissu musculaire squelettique en myoblastes.

21. Utilisation in vitro du milieu de culture selon la revendication 18 ou 19, pour l'obtention d'une population cellulaire comprenant un type cellulaire dominant choisi parmi les cellules CD34+, les cellules CD15+, les cellules CD56+, les cellules HLA Classe 1, ledit type cellulaire étant dominant lorsque la proportion de ce type cellulaire dépasse 50%.

22. Utilisation selon la revendication 21, **caractérisée en ce que** ledit type cellulaire dominant est choisi parmi les cellules CD34+, les cellules CD15+, les cellules CD56+.

23. Utilisation in vitro du milieu de culture selon la revendication 18 ou 19, pour la production d'un produit de thérapie cellulaire comprenant une population de cellules comprenant des cellules CD56+ comme type cellulaire dominant, ledit type cellulaire étant dominant lorsque la proportion de ce type cellulaire dans ladite population de cellules dépasse 50%.

## Claims

1. A method for preparing a composition intended for cell therapy in humans, said method comprising the following steps:
a) mincing a skeletal muscle tissue biopsy;
b) enzymatic dissociation of the muscle fibres and cells and separating the individualized cells by filtering;
c) culturing the cells obtained in step b) in a reactor for culturing adherent cells in the presence of a medium comprising MCDB 120 and D-valine substituted for L-valine until a population of cells comprising CD56+ cells as the dominant cell type is obtained, said cell type being dominant when the proportion of this cell type in said cell population exceeds 50%, said culture if necessary comprising one or more expansion phases;
d) harvesting the cell population obtained in step c);
e) if necessary, freezing the cell population harvested in step d).

2. A method according to claim 1, **characterized in that** the harvested cell population comprises 50 x 10⁶ cells to 800 x 10⁹ cells, preferably at least 500 x 10⁶ cells, among which myoblast cells expressing the CD56 marker constitute said dominant cell type.

3. A method according to claim 2, **characterized in that** the harvested cell population comprises at least 50%, preferably 60%, more preferably 70% of myoblast cells expressing the CD56 marker.

4. A method according to claim 2 or claim 3, **characterized in that** said medium contains a glucocorticoid and bFGF.

5. A method according to any one of claims 2 to 4, **characterized in that** the composition is intended for reconstituting skeletal, cardiac or visceral muscle tissue in humans.

6. A method according to any one of claims 2 to 5, **characterized in that** the composition is intended for the treatment of post-ischemic cardiac insufficiency in humans.

7. A method according to any one of claims 2 to 6, **characterized in that** the composition is intended for the treatment of innate or acquired muscular dystrophy.

8. A method according to any one of claims 2 to 7, **characterized in that** said mincing step is assisted by mechanical or electrical blades.

9. A method according to any one of claims 2 to 8, **characterized in that** it further comprises a step for CD34+ cell depletion.

10. A cell therapy product for its use in the treatment of a cardiac pathology in humans, comprising a population of 500 x 10⁶ to 800 x 10⁹ cells, said population comprising at least 50% of CD56+ cells.

11. A cell therapy product according to claim 10, **characterized in that** said population comprises at least 60% CD56+ cells.

12. A cell therapy product according to claim 10, **characterized in that** said population comprises at least 70% CD56+ cells.

13. A cell therapy product according to any one of claims 10 to 12, **characterized in that** said treatment of a cardiac pathology is a treatment by repair of cardiac tissue in humans.

14. A cell therapy product according to any one of claims 10 to 13, **characterized in that** said cardiac pathology is post-ischemic cardiac insufficiency.

15. A cell therapy product according to claim 14, **characterized in that** said post-ischemic cardiac insufficiency treatment is carried out concomitantly with a treatment by an angiotensin conversion enzyme inhibitor (ACEI).

16. A cell therapy product according to any one of claims 10 to 15, **characterized in that** said treatment of a cardiac pathology is a treatment by autologous transplantation.

17. Use of a cell population as defined in any one of claims 10 to 16, for the production of a cell therapy product intended for the treatment of a cardiac pathology.

18. A culture medium comprising MCDB 120 and D-valine substituted for L-valine.

19. A culture medium according to claim 18, which does not comprise phenol red and which does not comprise thymidine.

20. In vitro use of a culture medium according to claim 18 or claim 19, for the differentiation of skeletal muscle tissue cells into myoblasts.

21. In vitro use of a culture medium according to claim 18 or claim 19, for the production of a cell population comprising a dominant cell type selected from CD34+ cells, CD15+ cells, CD56+ cells, HLA class 1 cells, said cell type being dominant when the proportion of this cell type exceeds 50%.

22. Use according to claim 21, **characterized in that** said dominant cell type is selected from CD34+ cells, CD15+ cells and CD56+ cells.

23. In vitro use of a culture medium according to claim 18 or claim 19, for the production of a cell therapy product comprising a cell population comprising CD56+ cells as the dominant cell type, said cell type being dominant when the proportion of said cell type in said population exceeds 50%.

## Patentansprüche

1. Verfahren zur Herstellung einer Zusammensetzung für die Zelltherapie beim Menschen, wobei das Verfahren die folgenden Schritte aufweist:
a) Schneiden einer Skelettmuskelgewebe-Biopsie in dünne Scheiben,
b) enzymatisches Dissoziieren der Fasern und Muskelzellen und Trennung der individualisierten Zellen mittels Filtern,
c) Kultivieren der in Schritt b) erhaltenen Zellen in einem Kulturreaktor für adhärente Zellen in Gegenwart eines Mediums, das MCDB 120 und D-Valin, in Substitution von L-Valin aufweist, bis eine Zellpopulation erhalten wird, die die CD56+-Zellen als dominante Zellart aufweist, wobei die Zellart dominant ist, wenn der Anteil dieser Zellart in der Zellpopulation 50 % überschreitet, wobei die Kultur gegebenenfalls eine oder mehrere Expansionsphasen aufweist,
d) Ernten der in Schritt c) erhaltenen Zellpopulation,
e) gegebenenfalls Einfrieren der in Schritt d) geernteten Zellpopulation.

2. Verfahren gemäß Anspruch 1, **dadurch gekennzeichnet, dass** die geerntete Zellpopulation 50 x 10⁶ Zellen bis 800 x 10⁹ Zellen aufweist, vorzugsweise wenigstens 500 x 10⁶ Zellen, von denen die Myoblastzellen den Marker CD56 exprimieren, der die dominante Zellart bildet.

3. Verfahren gemäß Anspruch 2, **dadurch gekennzeichnet, dass** die geerntete Zellpopulation wenigstens 50 %, vorzugsweise 60 %, und noch bevorzugter 70 % Myoblastzellen aufweist, die den Marker CD56 exprimieren.

4. Verfahren gemäß 3, **dadurch gekennzeichnet, dass** das Medium Glucocorticoid und bFGF enthält.

5. Verfahren gemäß einem der Ansprüche 2 bis 4, **dadurch gekennzeichnet, dass** die Zusammensetzung für die Wiederherstellung von Skelettmuskel-, Kardial- oder Viszeralgewebe beim Menschen bestimmt ist.

6. Verfahren gemäß einem der Ansprüche 2 bis 5, **dadurch gekennzeichnet, dass** die Zusammensetzung für die Behandlung von post-ischämischer Herzinsuffizienz beim Menschen bestimmt ist.

7. Verfahren gemäß einem der Ansprüche 2 bis 6, **dadurch gekennzeichnet, dass** die Zusammensetzung für die Behandlung einer angeborenen oder erworbenen Muskeldystrophie bestimmt ist.

8. Verfahren gemäß einem der Ansprüche 2 bis 7, **dadurch gekennzeichnet, dass** der Schritt des in dünne Scheiben Schneidens mittels eines mechanischen oder elektrischen Schneidgranulators unterstützt wird.

9. Verfahren gemäß einem der Ansprüche 2 bis 8, **dadurch gekennzeichnet, dass** es des Weiteren einen Schritt der CD34+-Zelldepletion aufweist.

10. Zelltherapieprodukt für dessen Verwendung bei der Behandlung einer Herzerkrankung beim Menschen, das eine Population zu 500.10⁶ bis 800.10⁹ Zellen aufweist, wobei die Population wenigstens 50 % CD56+-Zellen aufweist.

11. Zelltherapieprodukt gemäß Anspruch 10, **dadurch gekennzeichnet, dass** die Population wenigstens 60 % CD56+-Zellen aufweist.

12. Zelltherapieprodukt gemäß Anspruch 10, **dadurch gekennzeichnet, dass** die Population wenigstens 70 % CD56+-Zellen aufweist.

13. Zelltherapieprodukt gemäß einem der Ansprüche 10 bis 12, **dadurch gekennzeichnet, dass** die Behandlung einer Herzerkrankung eine Behandlung durch Reparieren eines Herzgewebes beim Menschen ist.

14. Zelltherapieprodukt gemäß einem der Ansprüche 10 bis 13, **dadurch gekennzeichnet, dass** die Herzerkrankung eine post-ischämische Herzinsuffizienz ist.

15. Zelltherapieprodukt gemäß Anspruch 14, **dadurch gekennzeichnet, dass** die Behandlung einer post-ischämischen Herzinsuffizienz gleichzeitig mit einer Behandlung mit einem Angiotensin-Converting-Enzyminhibitor (ACEI; ACE-Hemmer) durchgeführt wird.

16. Zelltherapieprodukt gemäß einem der Ansprüche 10 bis 15, **dadurch gekennzeichnet, dass** die Behandlung einer Herzerkrankung eine Behandlung durch autologe Transplantation ist.

17. Verwendung einer Zellpopulation wie in einem der Ansprüche 10 bis 16 definiert für die Herstellung eines Zelltherapieprodukts, das zur Behandlung einer Herzerkrankung bestimmt ist.

18. Kulturmedium, das MCDB 120 und D-Valin, durch L-Valin substituiert, aufweist.

19. Kulturmedium gemäß Anspruch 18, das kein Phenolrot und kein Thymidin aufweist.

20. Verwendung *in vitro* des Kulturmediums gemäß Anspruch 18 oder 19 für die Differenzierung der Skelettmuskelgewebezellen in Myoblasten.

21. Verwendung *in vitro* des Kulturmediums gemäß Anspruch 18 oder 19, um eine Zellpopulation zu erhalten, die eine dominante Zellart aufweist, die aus den CD34+-Zellen, den CD15+-Zellen, den CD56+-Zellen, den HLA-Zellen Klasse 1 gewählt ist, wobei die Zellart dominant ist, wenn der Anteil dieser Zellart in der Zellpopulation 50 % überschreitet.

22. Verwendung gemäß Anspruch 21, **dadurch gekennzeichnet, dass** die dominante Zellart aus den CD34+-Zellen, den CD15+-Zellen, den CD56+-Zellen gewählt ist.

23. Verwendung *in vitro* des Kulturmediums gemäß Anspruch 18 oder 19 für die Herstellung eines Zelltherapieprodukts, das eine Zellpopulation aufweist, die CD56+-Zellen als dominante Zellart aufweist, wobei die Zellart dominant ist, wenn der Anteil dieser Zellart in der Zellpopulation 50 % überschreitet.
